(19) Europäisches Patentamt
European Patent Office
Office européen des brevets

(11) Veröffentlichungsnummer: **0 149 172**
**B1**

# (12) EUROPÄISCHE PATENTSCHRIFT

(45) Veröffentlichungstag der Patentschrift: **07.12.88**

(21) Anmeldenummer: **84115668.0**

(22) Anmeldetag: **18.12.84**

(51) Int. Cl.⁴: **C 07 C 87/30**, C 07 D 213/04, C 07 D 213/20, C 07 C 143/74, C 07 C 149/24, C 07 C 91/26, C 07 C 103/54, C 07 C 53/126, C 07 C 65/10, C 07 C 53/15, C 07 C 143/02

## (54) Fluorierte quaternäre Ammonium-Verbindungen, Verfahren zu deren Herstellung und deren Verwendung als Strömungsbeschleuniger.

(30) Priorität: **29.12.83 DE 3347378**

(43) Veröffentlichungstag der Anmeldung: **24.07.85 Patentblatt 85/30**

(45) Bekanntmachung des Hinweises auf die Patenterteilung: **07.12.88 Patentblatt 88/49**

(84) Benannte Vertragsstaaten: **DE**

(73) Patentinhaber: **HOECHST AKTIENGESELLSCHAFT, Postfach 80 03 20, D-6230 Frankfurt am Main 80 (DE)**

(72) Erfinder: **Ohlendorf, Dieter, Dr., Am Kühlen Grund 4, D-6237 Liederbach (DE)**
Erfinder: **Interthal, Werner, Dr., Dr.-Ludwig-Opel-Strasse 62, D-6090 Rüsselsheim (DE)**
Erfinder: **Bathelt, Heinrich, Dr., Beckstrasse 21, D-8262 Altötting (DE)**
Erfinder: **Hoffmann, Heinz, Prof. Dr., Waldsteinring 40, D-8580 Bayreuth (DE)**

(56) Entgegenhaltungen:
**EP-A- 0 097 926**
**AT-B- 276 336**
**CH-A- 509 966**
**CH-A- 525 855**
**DE-A- 1 912 130**
**DE-A- 1 966 931**
**DE-A- 2 119 302**
**DE-A- 2 357 916**
**DE-B- 1 006 426**
**DE-B- 1 643 561**
**DE-B- 2 244 297**
**DE-C- 571 294**
**DE-C- 845 515**
**FR-A- 1 534 666**
**FR-A- 2 084 888**
**GB-A- 758 156**

(56) Entgegenhaltungen: (Fortsetzung)
**GB-A- 906 409**
**US-A- 4 016 894**

**NATURE, Band 214, 1967, London; A. WHITE "Flow characteristics of complex soap systems", Seiten 585, 586**

EP 0 149 172 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Es ist allgemein bekannt, dass turbulent strömende Flüssigkeiten an den sie begrenzenden Wänden einen Reibungswiderstand erfahren. Es ist auch bekannt, dass man diesen Reibungswiderstand durch Zusatz geringer Mengen bestimmter Stoffe herabsetzen kann. Stoffe, die diese Wirkung zeigen, werden im englischen Sprachgebrauch als «drag reducing agents» bezeichnet. Im deutschsprachigen Raum wird für diese Stoffe die Bezeichnung «Strömungsbeschleuniger» verwendet (im folgenden abgekürzt durch SB). Unter einem Strömungsbeschleuniger versteht man danach einen Stoff, der in geringerer Menge einer turbulent oder pulsierend strömenden Flüssigkeit zugesetzt, diese Flüssigkeit – unter sonst gleichen Bedingungen – schneller strömen lässt. Strömungsbeschleuniger bewirken, dass man mit einer gegebenen Pumpe durch eine gegebene Rohrleitung mehr Flüssigkeit fördern kann.

In vielen Fällen ist allein schon diese Tatsache ein technischer Gewinn, wenn z.B. eine Rohrleitung im Normalbetrieb voll ausgelastet ist und zu bestimmten Zeiten ein Spitzenverbrauch zu fördern wäre. Da mit einer gegebenen Pumpleistung bei der Verwendung von Strömungsbeschleunigern mehr Flüssigkeit gefördert werden kann, wird auch in vielen Fällen die damit verbundene Energieeinsparung einen technischen Vorteil bringen. Schliesslich kann man, wenn man die Durchsatzmenge nicht vergrössern will, bei Verwendung von SB den Druckverlust herabsetzen oder Rohre mit kleinerem Querschnitt verwenden. Beides sind Massnahmen, die die Wirtschaftlichkeit im Betrieb einer Rohrleitung verbessern können.

Als Strömungsbeschleuniger für Wasser oder wässrige Lösungen sind neben hochmolekularen Verbindungen, wie Polyethylenoxid und Polyacrylamid, Lösungen von einigen Tensiden bekannt. Zusätze hochmolekularer Verbindungen besitzen jedoch nur eine beschränkte praktische Einsatzfähigkeit als Strömungsbeschleuniger, da sie in Bereichen hoher Scher- und Dehnbeanspruchung, wie z.B. in Pumpen oder in geringem Mass in der turbulenten Grenzschicht nahe der Rohrwandung, durch mechanischen Abbau irreversibel ihre Wirksamkeit als Strömungsbeschleuniger verlieren. Für geschlossene Wasserkreisläufe, in denen die selbe wässrige Lösung durch ein Rohrleitungssystem ständig umgepumpt wird, sind hochmolekulare Zusätze folglich ungeeignet, da der irreversible mechanische Abbau eine laufende Nachdosierung mit wirksamer hochmolekularer Substanz erforderlich macht.

Zusätze von Tensiden in Wasser weisen bekanntlich nicht den Nachteil des irreversiblen mechanischen Abbaues auf (US-PS 3 961 639). Zwar lässt sich auch hier in Bereichen sehr hoher Dehn- und Scherbeanspruchung, wie z.B. in Pumpen, ein mechanischer Abbau beobachten, der jedoch völlig reversibel ist, sobald die Lösung diese Bereiche passiert hat. So ist die strömungsbeschleunigende Wirkung einer wässrigen Lösung von Na-Oleat bei Zusatz von KCl + KOH oder NaCl + NaOH von Savins beschrieben (Reol. Acta 6, 323 (1967)). Asslanow et al. Akad. Nauk. SSSr. Mekh. Zhidk. Gaza 1, 36–43 (1980)) untersuchten u.a. wässrige Lösungen von Na-Laurat, -Myristat, -Palmitat und -Stearat bei pH = 11 als SB.

Chang et al. (US-PS 3 961 639) beschrieben die strömungsbeschleunigende Wirkung wässriger Lösungen einiger nicht ionischer Tenside mit Fremdelektrolytzusatz bei Temperaturen im Bereich des Trübungspunktes.

Wesentliche Nachteile der genannten Tensidlösungen sind ihre relativ hohen Einsatzkonzentrationen von mindestens 0,25 Gew.-%, die Bildung unlöslicher Seifen mit $Ca^{2+}$ und anderen Kationen, die Ausbildung zweier Phasen, die sich bei längerem Stehen trennen und zu Verstopfungen führen können, die Notwendigkeit der Zugabe von korrosionsfördernden Fremdelektrolyten, sowie ein sehr enger Temperaturbereich von wenigen Grad Celsius, in dem SB-Wirkung auftritt. Ebenfalls bekannt ist, dass wässrige Lösungen einiger kationischer Tenside, wie z.B. Cetylpyridiniumbromid (Inzh. Fizh. Zh. 38, No. 6, 1031–1037 (1980)) oder Cetyltrimethylammoniumbromid (Nature 214, 585–586 (1967)) in jeweils 1 : 1 molarer Mischung mit α-Naphthol als SB wirksam sind. Neben der schlechten Wasserlöslichkeit des α-Naphthols ist hier als entscheidender Nachteil zu nennen, dass derartige Mischungen ihre Wirksamkeit als SB innerhalb weniger Tage durch chemischen Abbau verlieren (US-PS 3 961 639, Conference Proceding: Intern. Conference on Drag Reduction, 4.–6.9.1974 Rolla Missouri, USA).

Bekannt ist auch die Verwendung von Cetyltrimethylammoniumsalicylat und Tetradecyltrimethylammoniumsalicylat als SB (WO 83/01583). Nachteil dieser Tensidsalze sowie aller bisher bekannten Tensidlösungen ist, dass sie oberhalb von ca. 80 °C ihre Wirksamkeit als SB verlieren und somit ungeeignet sind für Fernwärmenetze. Ein weiterer Nachteil der bekannten Tensidlösungen ist, dass sie in Gegenwart von Kohlenwasserstoffen, wie z.B. Schmieröle oder Motoröle, oder allg. fetthaltige Schmierstoffe die durch Pumpen und Ventile leicht in die Rohrleitungen gelangen können, ihre Wirksamkeit als SB bis auf einen geringen Rest oder gar völlig verlieren.

Überraschenderweise wurde nun gefunden, dass zum Unterschied zu allen bisher als SB bekannten Tensiden die nachfolgend aufgeführten Verbindungen in reiner Form auch ohne jegliche Zusätze in wässriger Lösung schon in kleinsten Konzentrationen auch in Gegenwart von Kohlenwasserstoffen, wie Schmier- und Motorölen, als Strömungsbeschleuniger wirksam sind. Weiterhin wurde gefunden, dass die Verbindungen auch bei Dauerbeanspruchung über Wochen keine Abnahme ihrer Effektivität als SB zeigen und dass einige Verbindungen sogar über 100 °C als SB wirksam sind.

Gegenstand der Erfindung sind neue quaternäre Ammoniumverbindungen der Formel

$$R–K^+A^-$$

wobei R n-Fluoralkyl, n-Fluoralkenyl, n-Alkyl, n-Alkenyl oder eine Gruppe der Formeln

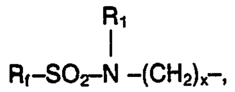 , 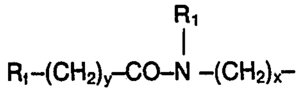

oder

$R_f-(CH_2)_yB(CH_2)_x-$

$R_f$ n-Fluoralkyl oder n-Fluoralkenyl, $R_1$ Wasserstoff, Methyl oder Ethyl, X eine ganze Zahl von 2 bis 6, y 0, 1 oder 2, B ein Sauerstoff- oder Schwefelatom,

$K^+$ ein Kation der Formeln

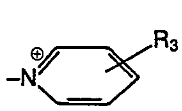 oder 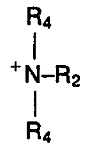

$R_2$ Wasserstoff, $C_1$–$C_4$-Hydroxyalkyl, $C_1$–$C_5$-Alkyl, Benzyl oder Phenyl, $R_3$ Wasserstoff, Methyl oder Ethyl, $R_4$ jeweils gleich oder verschieden sein können, Wasserstoff oder $C_1$–$C_3$-Alkyl und $A^-$ für den Fall der fluorierten Gruppen unter der Bedeutung von R ein n-Alkyl-, n-Alkenyl- oder n-Fluoralkylsulfonat-Anion, ein n-Alkyl-, n-Alkenyl- oder n-Fluoralkenylcarboxylat-Anion, ein Benzoat-, Phenylsulfonat-, Naphthoat-, Rhodanid-Anion oder ein Anion der Formel $C_n Cl_{2n+1}$ COO– mit n = 1, 2 oder 3 und $A^-$ für den Fall der nichtfluorierten Gruppe unter der Bedeutung von R ein n-Fluoralkyl- oder n-Fluoralkenylcarboxylat-Anion oder ein n-Fluoralkylsulfonat- oder n-Fluoralkenylsulfonat-Anion bedeuten, wobei die Verbindungen ausgenommen sind für die Fälle R = Fluoralkyl, Fluoralkenyl und $K^+$ Trialkylammonium und $A^-$: Alkylsulfonat, Alkenylsulfonat, Phenylsulfonat, Acetat; R = Alkyl und $K^+$ = $N^+H_3$ und $A^-$ = Fluoralkylcarboxylat und R–$K^+$ = ($C_1$–$C_2$)-Trialkylammonium, $A^-$ = Fluoralkylsulfonat und Fluoralkylcarboxylat, ebenso die Salze der Formel

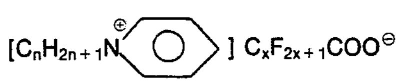

für n = 12–14
und x = 1–3.

Bevorzugt sind hierbei solche Verbindungen der Formel R–$K^+A^-$ wobei R $C_8$–$C_{14}$-n-Fluoralkyl, $C_8$–$C_{14}$-n-Fluoralkenyl, $C_1$–$C_{24}$-n-Alkyl oder $C_1$–$C_{24}$-n-Alkenyl, oder eine Gruppe der Formeln

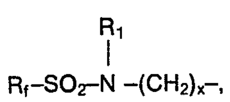 , 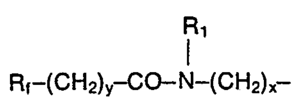

oder

$R_f-(CH_2)_yB(CH_2)_x-$

$R_f$ $C_8$–$C_{14}$-n-Fluoralkyl oder $C_8$–$C_{14}$-n-Fluoralkenyl, $R_1$ Methyl oder Ethyl, X eine ganze Zahl von 2 bis 6, y 0, 1 oder 2, B ein Sauerstoff- oder Schwefelatom, $K^+$ ein Kation der Formel

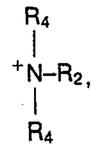

$R_2$ Wasserstoff, $C_1$–$C_3$-Hydroxyalkyl, Methyl oder Ethyl, $R_4$ Wasserstoff oder Methyl oder Ethyl und $A^-$ für den Fall der fluorierten Gruppen unter der Bedeutung von R ein $C_5$–$C_8$-n-Alkyl- oder n-Alkenylsulfonat-Anion, ein Trifluormethyl- oder Pentafluorethylsulfonat-Anion, ein $C_6$–$C_8$-n-Alkyl- oder n-Alkenylcarboxylat-Anion, ein $C_1$–$C_4$-n-Perfluoralkyl- oder $C_1$–$C_4$-n-Perfluoralkenylcarboxylat-Anion, ein Anion 25 der Formel

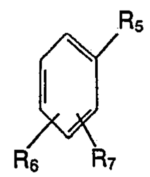 oder 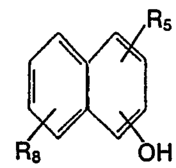

$R_5$–$COO^-$ oder –$SO_3^-$, $R_6$ $C_1$–$C_5$-Alkyl, $C_2$–$C_5$-Alkenyl oder $C_1$–$C_5$-Alkoxy in den Stellungen 3, 4 oder 5 zu $R_5$, oder $R_6$ Wasserstoff, wenn $R_7$ Hydroxy ist, $R_7$ Wasserstoff oder Hydroxy in den Stellungen 2 oder 3 zu $R_5$ oder $R_7$ $NO_2^-$, $F^-$, $Cl^-$, $Br^-$ oder $J^-$ in der Stellung 3 zu $R_5$, $R_8$ Wasserstoff oder Methyl, oder $A^-$ ein Rhodanid-Anion oder ein Anion der Formel $C_nCl_{2n+1}COO^-$ mit n = 1, 2 oder 3 und $A^-$ für den Fall der nicht-fluorierten Gruppen unter der Bedeutung von R ein n-Fluoralkyl- oder n-Fluoralkenyl-carboxylat-Anion, wobei die Summe von (2x + n) eine ganze Zahl von 19 bis 24 betragen soll, wenn x die Anzahl der C-Atome im Carboxylat-Anion und n die Anzahl der C-Atome in der Gruppe R ist; ein n-Fluoralkyl- oder n-Fluoralkenylsulfonat-Anion, wobei die Summe von (2y + n) 17 oder 18 betragen soll, wenn y die Anzahl der C-Atome im Sulfonat-Anion und n die Anzahl der C-Atome in der Gruppe R ist.

Besonders bevorzugt sind die Verbindungen der Formel R–$K^+$A wobei R–$K^+$ ein Kation der Formel

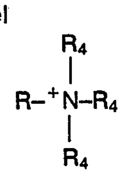 oder 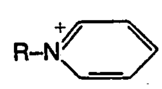

R eine Gruppe der Formeln $C_nF_{2n+1}$–$C_aH_{2a}$–, $C_nF_{2n}$–$C_{2a-1}$–, $C_nF_{2n+1}$–$CONR_1C_kH_{2k}$– oder $C_nF_{2n+1}$–$C_2H_4SC_kH_{2k}$–, $C_nF_{2n+1}$ $SO_2NR_1C_kH_{2k}$–,

n eine Zahl von 2 bis 10, a eine Zahl von 2 bis 20, k eine Zahl von 2 bis 6, wobei die Summe (2n+a) und (2n+k) eine Zahl von 12 bis 24 betragen soll, $R_4$ Methyl oder Ethyl, $R_4'$ Methyl, Ethyl, Hydroxymethyl oder Hydroxyethyl, $R_1$ Methyl oder Ethyl,

$A^-$ ein 2-Hydroxyphenylsulfonat-, m-Halogenbenzoat- oder Salicylat-Anion, ein Anion der Formel

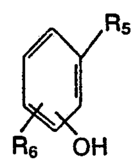

worin $R_5$ $COO^-$ oder $SO_3^-$ und $R_6$ $C_nH_{2n+1}-$ oder $C_nH_{2n}-$ oder $C_nH_{2n+1}-O-$ mit n einer Zahl von 1 bis 4 in den Stellungen 3, 4 oder 5 zu $R_5$ bedeuten oder $A^-$ eine 2-Hydroxy-1-naphthoat-, 3- oder 4-Hydroxy-2-naphthoat-, 2-Hydroxy-naphthalin-1-sulfonat-, 3- oder 4-Hydroxy-naphthalin-2-sulfonat-Anion, oder $A^-$ ein Anion der Formel $CF_3SO_3^-$ oder $n-C_xH_{2x+1}-SO_3^-$, wobei x 6 ist für den Fall (2n+a) mindestens 18, x ist 7 für den Fall (2n+a) mindestens 16 oder X ist 8 für den Fall (2n+a) eine Zahl von 14 bis 18 oder $A^-$ ist ein Anion der Formel $n-C_xF_{2x+1}-COO^-$, wobei x 2 oder 3 ist, wenn (2n+a) eine Zahl von 16 bis 22 ist oder X ist 4 oder 5, wenn (2n+a) eine Zahl von 14 bis 20 ist oder x ist eine Zahl von 6 bis 9, wenn (2n+1) eine Zahl von 14 bis 22 ist, oder $A^-$ ein Anion der Formeln $SCN^-$, oder $CCl_3COO^-$ bedeutet für den Fall n mindestens 6 und (2n+a) mindestens 16.

Besonders bevorzugt sind auch die Verbindungen der Formel $R-K^+A^-$, wobei $R-K^+$ ein Kation der Formel

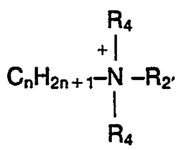 oder 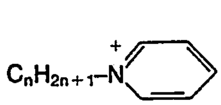

n eine Zahl von 1 bis 22, $R_4$ Methyl oder Ethyl, $R_2'$ Wasserstoff, Methyl, Ethyl, Hydroxymethyl oder Hydroxyethyl, $A^-$ ein Anion der Formeln $n-C_2F_{2x+1}-COO^-$, wobei x 2 oder 3 ist, wenn n+2x eine Zahl von 20 bis 28 ist, oder x ist 4 oder 5, wenn n+2x eine Zahl von 20 bis 26 ist, oder x ist eine Zahl von 6 bis 9, wenn (n+2x) eine Zahl von 19 bis 22 ist, oder $A^-$ ein Anion der Formel $n-C_xF_{2x+1}-SO_3^-$, wobei x eine Zahl ist, die so gewählt ist, dass die Summe (2x+n) 17 oder 18 beträgt.

Von besonderem Interesse sind die Verbindungen der Formel $(C_8F_{17}SO_3^-)(R_4-N(R_1,R_2,R_3))$, wobei $R_1$ Methyl oder Ethyl, $R_2$ Methyl oder Ethyl, $R_3$ Wasserstoff, Ethyl oder Methyl und $R_4$ Wasserstoff, Methyl oder Ethyl bedeuten sowie der Formel $(C_nH_{2n+1}{}^+NH_3)(C_xF_{2x+1}COO^-)$ bedeutet, wobei n eine Zahl von 14 bis 18 und x eine Zahl von 1 bis 3, insbesondere 1 bedeuten.

Die erfindungsgemässen Verbindungen können wie folgt hergestellt werden. Bei Perfluoralkylgruppen enthaltenden Ammoniumjodiden geht man zweckmässigerweise von den entsprechenden Dimethylaminen bzw. Amidaminen mit Dimethylamin-Strukturen aus und quaternisiert mit Methyljodid. Die Umsetzung kann in niederen Alkoholen wie Methanol, Ethanol, Isopropanol und ähnl. als Lösungsmittel erfolgen. Die entstandenen Trimethylammoniumjodide sind in diesem Falle in den genannten Alkoholen löslich. Die Quaternisierung kann jedoch auch in aprotischen Lösungsmitteln wie $CCl_4$, $CHCl_3$, $CH_2Cl_2$ oder 1.1.2-Trichlor-1.2.2-trifluorethan durchgeführt werden. Die Reaktion verläuft unter Rückflusstemperaturen des jeweiligen Lösungsmittels. Um die Reaktionsgeschwindigkeit zu erhöhen ist es jedoch zweckmässig, die Temperatur bei 100 °C oder 120 °C durchzuführen, wobei jedoch wegen der niedrigen Siedetemperatur des Methyljodids unter Druck gearbeitet werden muss.

Hexahydro- bzw. Oktahydroperfluoralkyltrimethylammoniumjodid der allgemeinen Formel

$$[R_F\ (CH_2)_n\ N^{\oplus}(CH_3)_3]\ J^{\ominus}\quad n = 3, 4$$

können auch durch direkte Alkylierung von Trimethylamin mit den entsprechenden Perfluoralkylgruppen enthaltenden Jodiden hergestellt werden.

Sollen die gewünschten Perfluoralkyltrimethylammoniumcarboxylate hergestellt werden, ist es zweckmässig, zunächst die Silbersalze der betreffenden Carbonsäure herzustellen. Dazu wird die Carbonsäure mit einer stöchiometrischen Menge an Alkali versetzt. Zu dem im Wasser löslichen Alkalisalz wird Silbernitrat-Lösung gegeben. Das sich bildende Silbercarboxylat ist wenig wasserlöslich, fällt aus und kann abgesaugt und getrocknet werden.

Die Herstellung der Perfluoralkyltrimethylammoniumcarboxylate erfolgt am besten in wasserfreien Lösungsmitteln wie Methanol, Ethanol oder Isopropanol durch Zusammengeben stöchiometrischer Mengen an Silbercarboxylat und Perfluoralkyltrimethylammoniumhalogenid bei Temperaturen von 50–60 °C. Nach Abkühlen wird vom gebildeten Silberhalogenid abgesaugt und durch Eindampfen des Filtrats das gewünschte Perfluoralkylgruppen enthaltende Trimethylammoniumcarboxylat gewonnen. Eine weitere Reinigung kann durch Umkristallisieren aus Lösungsmitteln wie Essigsäureethylester, Aceton, Acetonitril erfolgen.

In analoger Weise lassen sich aus den Perfluoralkyltrialkylammoniumhalogeniden durch Umsetzung mit entsprechenden Silbersalzen die anderen, unter der Bedeutung von $A^+$ aufgeführten Anionen einführen.

Eine weitere Herstellmöglichkeit für die gewünschten Perfluoralkylgruppen enthaltenden Trimethylammoniumcarboxylate besteht auch in der Gewinnung der Alkyltrimethylammonium-Hydroxidlösung durch Behandlung der Alkyltrimethylammoniumhalogenide oder anderen Salzen mit einem stark basischen Anionenaustauscher, was in einem wasserfreien Lösungsmittel wie Methanol oder Ethanol durchzuführen ist. Anschliessend wird mit der gewünschten Carbonsäure neutralisiert und das Salz aus der Lösung durch Eindampfen gewonnen.

Die quartären Ammoniumverbindungen, in denen $K^+$ ein Pyridinium-Ion bedeutet, lassen sich herstellen durch Umsetzung von Verbindungen der Formel R–J mit Pyridin b zu substituierten Pyridinen.

Gegenstand der Erfindung ist auch die Verwendung von quarternären Ammoniumverbindungen der Formel

$$R-K^+A^-$$

als Strömungsbeschleuniger zur Verminderung des Reibungswiderstandes von wässrigen Medien, wobei R n-Fluoralkyl, n-Fluoralkenyl, n-Alkyl, n-Alkenyl oder eine Gruppe der Formel

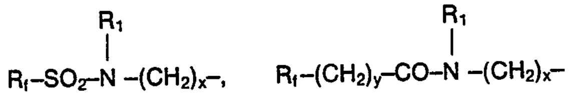

oder

$R_f-(CH_2)_yB(CH_2)_x-$

$R_f$ n-Fluoralkyl oder n-Fluoralkenyl, $R_1$ Wasserstoff, Methyl oder Ethyl, X eine ganze Zahl von 2 bis 6, y 0, 1 oder 2 ein Sauerstoff- oder Schwefelatom,

$K^+$ ein Kation der Formeln

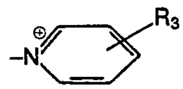

oder

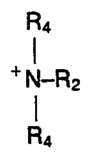

$R_2$ Wasserstoff, $C_1$–$C_4$-Hydroxyalkyl, $C_1$–$C_5$-Alkyl, Benzyl oder Phenyl, $R_3$ Wasserstoff, Methyl oder Ethyl, $R_4$ jeweils gleich oder verschieden sein können, Wasserstoff oder $C_1$–$C_3$-Alkyl und $A^-$ für den Fall der fluorierten Gruppen unter der Bedeutung von R ein n-Alkyl-, n-Alkenyl- oder n-Fluoralkylsulfonat-Anion, ein n-Alkyl-, n-Alkenyl- oder n-Fluoralkenylcarboxylat-Anion, ein Benzoat-, Phenylsulfonat-, Naphthoat-, Jodid-, Rhodanid-Anion oder ein Anion der Formel $C_nCl_{2n+1}COO^-$ mit n = 1, 2 oder 3 und $A^-$ für den Fall der nichtfluorierten Gruppe unter der Bedeutung von R ein n-Alkyl-, n-Alkenylcarboxylat-Anion, ein n-Fluoralkyl- oder n-Fluoralkenylcarboxylat-Anion oder ein n-Fluoralkylsulfonat- oder n-Fluoralkenylsulfonat-Anion bedeuten, wobei die Verbindungen ausgenommen sind für die Fälle R = Fluoralkyl und $K^+$ Trialkylammonium und $A^-$: Jodid; R = Alkyl und $K^+ = N^+H_3$ und $A^-$ = Fluoralkylcarboxylat und $R-K^+ = (C_1-C_2)$-Trialkylammonium, $A^-$ = Fluoralkylsulfonat und Fluoralkylcarboxylat, ebenso die Salze der Formeln

$[C_{16}H_{33}N(CH_3)_3]^+ \; C_nH_{2n+1}COO^-$

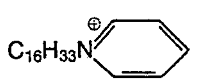 $C_nH_{2n+1}COO^-$,

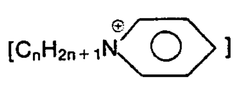 $C_xF_{2x+1}COO^{\ominus}$

für n = 12–14
und x = 1–3.

Diese Verbindungen werden den wässrigen Medien zugegeben in Konzentration von 0,01 bis 5 Gew.-%, bevorzugt von 0,05 bis 1 Gew.-%, besonders bevorzugt von 0,1 bis 0,5 Gew.-%. Für jedes Tensid existiert jedoch in Abhängigkeit von der Temperatur eine andere untere kritische Konzentrationsgrenze für eine ausreichende Wirkung als SB, die jedoch, wie weiter hinten beschrieben, durch einen einfachen Vorversuch bestimmt werden kann. Die Wirkung als SB ist abhängig von der Temperatur. Die genannten Verbindungen wirken insgesamt in einem Temperaturbereich von 0 °C bis 145 °C; ein einzelnes Tensid zeigt Wirksamkeit als SB jedoch nur über ein Temperaturintervall von ca. 45 °C (∓20 °C). Die untere Temperaturgrenze ist bei allen Tensiden die Löslichkeitstemperatur (isotrope Lösung) oder besser der Krafft-Punkt. Ist das Tensid jedoch in Lösung, so kann die Löslichkeitstemperatur, in den meisten Fällen, für einige Stunden bis Wochen um 5 bis 25 °C unterschritten werden, ohne dass ein Verlust der Wirksamkeit als SB auftritt. Eine Verwendung derjenigen Tenside als SB, die bis zum Schmelzpunkt des Wassers in Lösung bleiben, ist bei Temperaturen unter 0 °C möglich, wenn der Schmelzpunkt des Wassers durch Zumischung organischer Lösungsmittel, wie z. B. Ethylenglycol oder Isopropanol, erniedrigt wird. Eine Absenkung der Schmelztemperatur des Wassers durch Elektrolytzugabe, wie z. B. NaCl, ohne Verlust der Wirksamkeit als SB ist nur bedingt möglich. Besonders hervorzuheben ist, wie Beispiel 29 zeigt, dass eine Reihe der gesamten Tenside auch bei Temperaturen über 100 °C als SB wirksam ist.

Die Wirksamkeit der genannten Verbindungen als SB wurde nicht nur in entionisiertem Wasser (E-Wasser) bei einem pH-Wert von 5 bis 7 untersucht, sondern es wurden auch verschiedene Salze (Fremdelektrolyte), wie z. B. NaCl, $CaCl_2$, $AlCl_3$, $Na_2CO_3$, $CaCO_3$, $Na_3PO_4$ und andere zugesetzt oder auch der pH-Wert der Lösungen durch Zugabe von NaOH oder HCl variiert. Es zeigte sich, dass die Zugabe von Fremdelektrolyten von keiner Beeinflussung (siehe Beispiele 8, 9, 10 und 11) bis zu einer Verbesserung (siehe Beispiele 14, 15) der Wirksamkeit als SB führt; ein analoges Verhalten ergibt sich bei der Änderung des pH-Wertes. Die Menge an Fremdelektrolyt, die man der wässrigen Tensidlösung zugeben kann, ist nach oben begrenzt durch die Konzentration, bei der ein Aussalzeffekt für das Tensid auftritt, verbunden mit einer Abnahme oder dem völligen Verschwinden der Wirksamkeit als SB (vgl. Beispiele 8, 9, 10, 14, 15, 16). Die Höchstkonzentration wird auch durch den Fremdelektrolyten bestimmt und ist um so niedriger, je höher die Wertigkeit (Ladung) von Anion oder Kation ist.

Anstelle der Zugabe der erfindungsgemäss genannten Tenside kann man auch so vorgehen, dass man das Halogensalz des Kations $R_1-K^+ Hal^-$, wie z. B.

$(C_nF_{2n+1}N(CH_3)_3)$ Hal, 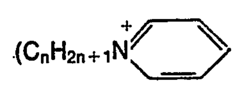 Hal,

$(C_nF_{2n+1}C_1H_{21}N(CH_3)_3)$ Hal,
$(C_nF_{2n} \; C_1H_{21}N(CH_3)_3)$ Hal,
$(C_nF_{2n+1}CONH \; C_2H_{21}N(CH_3)_3)$ Hal,
$C_nF_{2n+1}SO_2N(C_2H_5) \; C_1H_{21}N(CH_3)_3)$ Hal
oder
$(C_nF_{2n+1}CH_2CH_2SCH_2CH_2N(CH_3)_3)$ A
mit A = $Hal^-$ der $CH_3SO_4^-$
oder die entsprechenden Pyridiniumverbindungen mit Hal = Cl oder Br im molaren Verhältnis 1 : 1 gemischt mit einem Alkalisalz des Anions NaA, wie z. B. Natriumsalicylat oder andere Natrium-hydroxi-benzoate oder Natrium-3-hydroxi-2-naphthoat oder Natriumhydroxinaphthoate oder die Natriumsalze der genannten Carbon- und Sulfonsäuren als Strömungsbeschleuniger einsetzt. Die Wirkung ist dann gleich der Wirkung, die man mit den reinen Tensidsalzen unter Zusatz von Alkalihalogeniden erreicht. Auch Mischungen, die vom molaren Verhältnis 1 : 1 abweichen, wie z. B. 1 : 2, zeigen Wirkung als SB.

Die maximale Wirksamkeit als Strömungsbeschleuniger ist auch von der Zeit abhängig, die verstrichen ist seit der Herstellung der wässrigen Tensidlösungen. Die Tensidlösungen zeigen zwar bereits sofort nach dem Ansetzen der Lösungen eine Wirkung als Strömungsbeschleuniger, doch kann sich diese Wirkung während einer Woche noch deutlich ändern. Die Zeit, die benötigt wird, um eine konstante Wirkung zu erzielen, lässt sich für den Einzelfall unschwer durch einfache Versuche ermitteln. In den meisten Fällen wird eine konstante Wirkung nach 1 bis 3 Tagen, in wenigen Fällen nach einer Woche erreicht. Eine Änderung, d.h. Verbesserung oder Verschlechterung der Wirkung als SB tritt dann nicht mehr ein.

Von einigen Tensiden, wie z. B. dem Hexadecylpyridiniumsalicylat ist bekannt (H. Hoffmann et al., Ber. Bunsenges. Phys. Chem. 85 (1981) 255), dass sie ab einer ganz bestimmten, für jedes Tensid charakteristischen Konzentration, der $CMC_{II}$, grosse, nichtkugelförmige, meist stäbchenförmige Mizellen aus den einzelnen Tensidionen und Gegenionen aufbauen.

Überraschenderweise wurde nun gefunden, dass Tenside in wässriger Lösung immer dann als Strömungsbeschleuniger wirksam sind, wenn sie für Konzentrationen grösser der $CMC_{II}$ nichtkugelförmige, vorzugsweise stäbchenförmige Mizellen ausbilden. Nichtkugelförmige, vorzugsweise stäbchenförmige Mizellen liegen vor, wenn bei der Untersuchung der isotropen Tensidlösung mit Hilfe der Methode der elektrischen Doppelbrechung mit gepulstem, rechteckförmigen elektrischen Feld (E. Fredericq und C. Houssier, Eletric Dichroism and Eletric Birefringence, Claredon Press, Oxford 1973 und H. Hoffmann et al., Ber. Bunsenges. Phys. Chem. 85 (1981) 255) ein Messsignal gefunden wird, aus dessen Abfall sich eine Relaxationszeit von $\tau > 0{,}5\ \mu s$ bestimmen lässt. Die untere Konzentrationsgrenze, ab der ein Tensid in wässriger Lösung als Strömungsbeschleuniger wirksam ist, wird daher immer durch die $CMC_{II}$, vorzugsweise durch den 1,3 bis 2-fachen Konzentrationswert der $CMC_{II}$, festgelegt. Die Bestimmung der $CMC_{II}$ ist z.B. durch Messung der elektrischen Leitfähigkeit der Tensidlösung in Abhängigkeit von der Tensidkonzentration möglich, wie bei H. Hoffmann et al. (Ber. Bunsenges. Phys. Chem. 85 (1981) 255) beschrieben. Es zeigte sich, dass der Wert der $CMC_{II}$ temperaturabhängig ist und sich mit zunehmender Temperatur zu höheren Tensidkonzentrationen verschiebt.

Zur Festlegung der Tensidkonzentration, die minimal notwendig ist, um eine ausreichende Wirkung als SB in einem bestimmten Temperaturbereich zu erzielen, ist die Bestimmung der $CMC_{II}$ bei der Anwendungstemperatur mit Hilfe der elektrischen Leitfähigkeit ein geeigneter Vorversuch.

Die Untersuchung der genannten Tenside auf ihre Wirksamkeit als SB erfolgte in den meisten Fällen in der üblichen Art, indem für die jeweilige wässrige Lösung der Tenside der Druckabfall $\Delta P$ über die Strecke L bei Durchströmen eines Rohres mit dem Querschnitt d für verschiedene Strömungsgeschwindigkeiten u gemessen wurde. Aus diesen Werten lassen sich die dimensionslosen Grössen, Reibungsbeiwert $\lambda$ und die Reynoldszahl Re, berechnen,

$$\lambda = \frac{2\,d}{\delta u^2} \cdot \frac{\Delta P}{}$$

$$Re = \frac{u\,d}{\nu}$$

wobei $\delta$ die Dichte und $\nu$ die kinematische Viskosität bedeuten. Üblicherweise werden für $\delta$ und $\nu$ jeweils die entsprechenden Werte des reinen Lösungsmittels Wasser, eingesetzt. Die so erhaltenen Werte, $\lambda$, Re, für die untersuchten Tensid-Lösungen wurden in der üblichen doppelt logarithmischen Auftragung $\lambda$ gegen Re mit den entsprechenden Werten für reines Wasser, wiedergegeben durch

$$1/\sqrt{\lambda} = 2 \log Re \sqrt{\lambda} - 0{,}8$$

verglichen. Eine Wirkung als SB oder auch Reibungsminderung liegt dann vor, wenn gilt: $\lambda H_2O - \lambda SB > 0$, bzw. die Grösse der Reibungsminderung in Prozent berechnet sich nach:

$$\alpha = \%\ \text{Reibungsminderung} = \frac{\lambda H_2O - \lambda SB}{\lambda H_2O} \times 100$$

Wie aus Figur 1 ersichtlich ist, wirken die genannten Tensidlösungen als SB in der Weise, dass die prozentuale Reibungsminderung mit ansteigender Reynoldszahl zunimmt, dann aber nach Überschreiten einer bestimmten Reynoldszahl, $Re_{max}$, mit maximaler prozentualer Reibungsminderung, sehr schnell wieder abnimmt. Der Grad der Wirksamkeit einer Tensidlösung als SB soll im folgenden Text durch die Grösse von $Re_{max}$ gekennzeichnet werden; demnach besitzt eine Tensidlösung mit $Re_{max} = 20\,000$ eine bessere Wirksamkeit als SB als eine Tensidlösung mit $Re_{max} = 10\,000$. Der zugehörige $\alpha$-Wert soll mit $\alpha_{max}$ gekennzeichnet werden. Die entscheidende Grösse für das Maximum der Wirksamkeit ist jedoch nicht die Reynoldszahl sondern die Wandschubspannung $\tau_w$:

$$\tau_w \frac{\Delta P\,d}{4L} = \frac{\lambda}{8}\,\delta u^2 \quad ;$$

d.h. dass $Re_{max}$ für den gleichen SB bei verschiedenen Rohrdurchmessern unterschiedliche Werte einnimmt (vgl. Beispiel 7 und 28 oder Beispiele 17 und 29). Werden jedoch alle SB mit der gleichen

Apparatur mit gleichem Rohrdurchmesser untersucht, ist $Re_{max}$ eine geeignete Grösse für den Vergleich der Wirksamkeit der SB untereinander. Die Untersuchungen der Tensidlösungen ergaben häufig nur dann reproduzierende Ergebnisse, wenn die wässrigen Lösungen der Tensidsalze vor den Messungen jeweils für ca. 1 Woche bei den Messtemperaturen aufbewahrt wurden. Die Lösungen zeigen zwar auch sofort nach dem Ansetzen eine Wirkung als Strömungsbeschleuniger, die sich jedoch im Verlauf einer Woche noch deutlich ändern kann.

Die so behandelten Tenside wurden einer Vielzahl von Tests unterzogen. So ergaben Dauerversuche über viele Tage, wie aus Beispiel 28 ersichtlich, dass bei den aufgeführten Tensiden keine Abnahme ihrer strömungsbeschleunigenden Wirkung durch mechanischen oder chemischen Abbau auftritt. Weiterhin zeigte sich, dass die Wirksamkeit als SB der genannten Tenside mit zunehmender Konzentration zunimmt; allerdings steigt auch die Viskosität der Lösungen an, so dass die prozentuale Reibungsminderung bei kleineren Reynoldszahlen schlechter wird, wie aus Figur 1 zu entnehmen ist.

Die durchgeführten Untersuchungen zeigen, dass sich die genannten Tensid-Salze als Strömungsbeschleuniger überall dort eignen, wo Wasser durch Rohrleitungen gepumpt wird, insbesondere aber dort, wo Wasser in einem Rohrleitungssystem ständig im Kreislauf umgepumpt wird, wie z.B. in Kühl- und Heizkreisläufen, da hier eine hohe Langzeitstabilität des SB, wie sie die genannten Tensid-Salze aufweisen, unbedingt erforderlich ist. Zusätzlich zeigen einige der genannten Tenside eine Eignung insbesondere für Fernwärmenetze, da diese Tenside auch oberhalb von 90 °C bei Beanspruchung über Wochen (siehe Beispiel 28 und 29) ihre Wirkung als Strömungsbeschleuniger beibehalten.

Die Zudosierung der Tensid-Salze in das die Rohrleitungen durchströmende Wasser kann sowohl in Form einer konzentrierten Tensidlösung (1–10 Gew.-%) erfolgen, als auch durch Zugabe der reinen kristallinen Tensid-Salze. Wegen des guten Durchmischungseffektes ist eine Zudosierung in das Rohrleitungssystem kurz vor einer Pumpe der günstigste Ort.

Beispiel 1
1.1.2.2.3.3.4.4-Octahydroperfluordodecyltrimethylammoniumsalicylat

a) Herstellung von 1.1.2.2.3.3.4.4-Octahydroperfluordodecyl-trimethylammoniumjodid

100 g $C_8F_{17}(CH_2)_4J$ und 30 g bei −20 °C kondensiertes Trimethylamin wurden in einen 250 ml Edelstahlautoklaven eingefüllt und unter Eigendruck 10 Stunden lang bei 80 °C gerührt. Nach Abkühlen des Autoklaven wurde überschüssiges Trimethylamin entgast, das entstandene quartäre Ammoniumsalz mit heissem Methanol herausgelöst und filtriert. Das Filtrat wird am Rotationsverdampfer zur Trockne eingedampft. Ausbeute an Rohprodukt: 103,5 g = 96,9% d.Th. eines hellgelben Pulvers. Für die weitere Verarbeitung wurde das Produkt aus Aceton umkristallisiert.

b) Herstellung des Salicylats

48,4 g Salicylsäure und 14,0 g NaOH wurden in 100 ml Wasser gelöst. Diese Lösung wurde zu einer Lösung von 59,5 g Silbernitrat in 100 ml Wasser gegeben und eine halbe Stunde gut gerührt. Das abgeschiedene Silbersalicylat wird über eine Filternutsche abgesaugt und mit je 50 ml Wasser, Methanol und Aceton nachgewaschen. Es wird anschliessend im Exsiccator unter Vakuum getrocknet. Ausbeute 79 g = 92,1% d.Th.

In eine Lösung von 13,2 g des nach Beispiel 1a hergestellten Produkts der Formel

$$(C_8F_{17}(CH_2)_4N^{\oplus}(CH_3)_3)\ J^-$$

in 100 ml Methanol werden 4,9 g Silbersalicylat gegeben. Man erwärmt das Gemisch auf ca. 50 °C und saugt anschliessend vom ausgefallenen Silberjodid ab. Das klare, farblose Filtrat wird am Rotationsverdampfer eingedampft. Man erhält 11,8 g Rohprodukt als farbloses Pulver, das aus Aceton umkristallisiert wird. Schmp. 153 °C. Die Titration mit Perchlorsäure in Eisessig ergibt als Molgewicht 650 (ber. 671).

Die Leitfähigkeitsmessungen zeigen, dass die $CMC_I$ und $CMC_{II}$ für diese Verbindung zusammenfallen; die Werte betragen für 55 °C 270 ppm (0,42 mmol/l) und für 70 °C 375 ppm (0,58 mmol/l).

Beispiel 2
1.1.2-Trihydroperfluordodecenyltrimethylammoniumsalicylat

a) Herstellung von 1.1.2-Trihydroperfluordodecenyl-trimethylammoniumjodid

100 g

$$C_9F_{19}CF{=}CHCH_2N(CH_3)_2$$

(hergestellt nach US-Patent 3 535 381) wurden in 100 ml Diisopropylether gelöst und in einem 1 Liter Glasautoklaven mit 24,9 g Methyljodid versetzt. Nach 10-stündigem Rühren bei 80 °C unter Eigendruck wurde abgekühlt, das Gemisch am Rotationsverdampfer eingeengt, das ausgefallene quartäre Salz abgesaugt und getrocknet. Ausbeute 118,5 g = 94,5% d.Th. Für die weitere Umsetzung wurde das Produkt aus Aceton umkristallisiert.

b) Herstellung des Salicylats

Zu einer Lösung von 14,3 g

$$(C_9F_{19}CF{=}CHCH_2N^{\oplus}(CH_3)_3)\ J^-$$

In 50 ml Methanol wurden 4,9 g Silbersalicylat gegeben und das Gemisch unter Rühren auf 50 °C erwärmt. Nach 30 Minuten wird vom ausgefallenen AgJ abgesaugt, das klare Filtrat am Rotationsverdampfer bis zur Trockne eingedampft. Man erhält 12,8 g Rohprodukt, das sind 89,7% d.Th., als hellgelbes Pulver.

Das Produkt kann aus Aceton umkristallisiert werden. Schmp. 173 °C (Zers.). Die Titration mit Perchlorsäure in Eisessig ergibt als Molmasse 739 (ber. 723).

Die $CMC_{II}$-Werte, bestimmt durch Leitfähigkeitsmessungen, betragen:
25 °C 124 ppm (0,17 mmol/l), 55 °C 225 ppm (0,31 mmol/l) und 70 °C 645 ppm (0,87 mmol/l).

Beispiel 3
1.1.2-Trihydroperfluordecenyl-trimethylammonium-3-hydroxy-2-naphthoat.

a) Herstellung von 1.1.2-Trihydroxyperfluordecenyl-trimethylammoniumjodid
80 g

$$C_7F_{15}CF=CHCH_2N(CH_3)_2$$

wurden in 250 ml Diethylether gelöst, mit 24,1 g Methyljodid versetzt und anschliessend in einem 1 Liter Glasautoklaven 8 Stunden auf 80 °C erhitzt. Nach Abkühlen wird die Hälfte des eingesetzten Lösungsmittels am Rotationsverdampfer abdestilliert, das ausgefallene quartäre Salz wird abfiltriert und 2 mal mit je 50 ml Diethylether gewaschen. Ausbeute 97,3 g; das sind 93,3% d.Th. Für die weitere Verarbeitung wird das Produkt aus Aceton umkristallisiert.

b) Herstellung des 3-Hydroxy-2-naphthoats

65,9 g 2-Hydroxy-2-naphthoesäure wurden in 100 ml Methanol gelöst und 14,0 g NaOH, gelöst in 50 ml Wasser zugegeben. Unter Rühren wird langsam eine aus 59,5 g Silbernitrat und 100 ml Wasser hergestellte Lösung zugegeben. Es bildet sich das schwer lösliche Silbernaphthoat, das abgesaugt und je einmal mit 100 ml Wasser, Methanol und Aceton gewaschen wird.

Nach Trocknen im Vakuum erhält man 98 g = 94,9% d.Th. eines nahezu farblosen Salzes.

Zu einer Lösung von 9,2 g

$$(C_7F_{15}CF=CHCH_2N^{\oplus}(CH_3)_3)\ J^-$$

in 200 ml Methanol werden 4,4 g des vorher beschriebenen Silber-3-hydroxy-2-naphthoats gegeben. Man erhitzt kurz auf ca. 60 °C und saugt nach abkühlen vom ausgefallenen Silberjodid ab.

Das klare Filtrat wird am Rotationsverdampfer zur Trockne eingedampft, der Rückstand anschliessend aus Aceton umkristallisiert. Ausbeute 8,4 g, das sind 83% d. Theorie.

Diese Verbindung wurde mit Perchlorsäure in Eisessig titriert. Aus dem Verbrauch errechnete sich eine Molmasse von 653 (ber. 673).

Beispiel 4
1.1.2-Trihydroperfluordodecenyltrimethylammoniumperfluorpropionat.

In eine Lösung von 4,3 g des nach Beispiel 2a hergestellten

$$(C_9F_{19}CF=CHCH_2N^{\oplus}(CH_3)_3)\ J^-$$

in 100 ml Methanol wurden 5,4 g Ag-propionat eingerührt. Nach kurzem Erwärmen des Gemisches wird vom ausgefallenen Ag J abgesaugt, das klare Filtrat am Rotationsverdampfer eingeengt. Rohausbeute 14,2 g eines nahezu farblosen Pulvers, das aus einem Gemisch Aceton/1.1.2-Trichlor-1.2.2-trifluorethan im Volumenverhältnis 1 : 1 umkristallisiert werden konnte. Schmp. 148 °C.

Die Titration mit Perchlorsäure in Eisessig ergab eine Molmasse von 759 (ber. 749).

Die CMC-Werte, bestimmt durch Leitfähigkeitsmessungen, betragen: 55 °C $CMC_I$ 179 ppm (0,23 mmol/l), $CMC_{II}$ 700 ppm (0,92 mmol/l); 70 °C $CMC_I$ 250 ppm (0,33 mmol/l), $CMC_{II}$ 1100 ppm (1,5 mmol/l).

Beispiel 5
Cetyltrimethylammonium-perfluorbutyrat

Zu einer Lösung von 6,4 g Cetyltrimethylammoniumchlorid (im Handel erhältlich) in 50 ml Methanol wurden 6,4 g Silberperfluorbutyrat, (analog Beispiel 1b aus Perfluorbuttersäure und $AgNO_3$ hergestellt) gelöst 50 ml Methanol gegeben. Man erwärmt 10 Minuten auf etwa 60 °C, kühlt und saugt vom ausgefallenen Silberchlorid ab. Das Filtrat wird am Rotationsverdampfer vom Lösungsmittel befreit, der Rückstand, ein gelbes Pulver (14,2 g) wurden aus Aceton/1.1.2-Trichlor-1.2.2-trifluorethan umkristallisiert. Schmp. 152 °C.

Die Titration mit Perchlorsäure in Eisessig ergibt eine Molmasse von 506 (ber. 497).

Die CMC-Werte, bestimmt durch Leitfähigkeitsmessungen, betragen:
25 °C $CMC_I$ = $CMC_{II}$ 120 ppm (0,24 mmol/l); 55,5 °C $CMC_I$ 126 ppm (0,25 mmol/l); $CMC_{II}$ 370 ppm (0,73 mmol/l); 69,5 °C $CMC_I$ 207 ppm (0,41 mmol/l); $CMC_{II}$ 970 ppm (1,9 mmol/l).

Beispiel 6
Tetramethylammonium-perfluoroctylsulfonat

a) Herstellung von Tetramethylammoniumjodid

29,1 g auf −20 °C abgekühltes Trimethylamin wurden in einem gekühlten 250 ml Edelstahlautoklaven vorgelegt und mit 70 g Methyljodid, gelöst in 70 ml Methanol, versetzt. Nach Schliessen des Autoklaven wird dieser 10 Stunden bei 80 °C geschüttelt. Nach Abkühlen wird der Autoklav entspannt, der Inhalt mit heissem Methanol aufgenommen und filtriert. Das Filtrat wird am Rotationsverdampfer zur Trockne eingedampft. Man kristallisiert aus einem Gemisch Wasser/Methanol um.

b) Herstellung des Perfluoroctylsulfonats

In eine Lösung von 6,0 g Tetramethylammoniumjodid in 50 ml Methanol/Wasser im Volumenverhältnis 1 : 1 werden 18,2 g des analog Beispiel 1b hergestellten Silberperfluoroctylsulfonats, gelöst in 100 ml Wasser/Methanol (Volumenverhältnis 1 : 1) gegeben.

Nach kurzem Erwärmen (10 Min) auf 60 °C wird abgekühlt, vom ausgefallenen Silberjodid abfiltriert und getrocknet. Man erhält 16,6 g eines pulverförmigen, gelblichen Salzes, das aus einem

Gemisch Aceton/Methanol/1.1.2-Trichlor-1.2.2-trifluorethan (im Volumenverhältnis 1 : 1 : 1) umkristallisiert wird.

Für das Produkt wird – nach Titration mit Perchlorsäure in Eisessig – eine Molmasse von 586 gefunden (ber. 573).

Aus der Leitfähigkeitsmessung ergibt sich bei 40 °C $CMC_I = CMC_{II}$ 1180 ppm (1,1 mmol/l).

Beispiel 7

Von der in Beispiel 1 charakterisierten Verbindung

$(C_8F_{17}(CH_2)_4 \overset{\oplus}{N} (CH_3)_3)$ 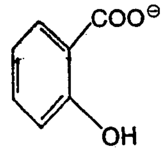

(abgekürzt: $(CF)_8$ $(CH)_4$ TA-Sal) wurde eine Konzentrationsreihe von 200, 500, 750, 1000, 1500 und 2000 Gew.-ppm in E-Wasser angesetzt, indem die entsprechenden Gewichtsmengen von 0,2; 0,5; 0,75; 1; 1,5 und 2 g des $(CF)_8$ $(CH)_4$ TA-Salzes mit E-Wasser auf 1000 g eingewogen wurden. Während des Auflösens (Löslichkeitstemperatur ca. 40 °C) wurden die Lösungen kurz auf ca. 90 °C erhitzt und nach dem Abkühlen auf 55 °C für 1 Woche ohne Rühren bei dieser Temperatur aufbewahrt.

Die Untersuchung auf Reibungsminderung erfolgte anschliessend in einem Turbulenzrheometer (Polymer Letters 9, 851 (1971)), indem analog zu einer Spritze eine Flüssigkeitsmenge von 1,5 l mit Hilfe eines Kolbens durch das Messrohr gedrückt wird. Die Bewegung des Kolbens wird während der Messung beschleunigt, so dass die gesamte Fliesskurve, wie in der Figur 1 dargestellt, in einer Messung erfasst wird. Der Durchmesser des Messrohres beträgt 3 mm, die Messstrecke für $\Delta P$ 300 mm und die Einlaufstrecke 1200 mm.

In dieser Apparatur wurde dieselbe Konzentrationsreihe bei 70 °C und 90 °C gemessen, nachdem die Lösung zuvor ebenfalls für 1 Woche bei 70 °C und 99 °C aufbewahrt worden war. Anschliessend wurden die Lösungen mit 200, 500, 750 und 1000 Gew.-ppm an $(CF)_8$ $(CH)_4$ TA-Salz auf Raumtemperatur (25 °C), d.h. auf 15° unterhalb der Löslichkeitstemperatur, abgekühlt, für 1 Woche bei dieser Temperatur aufbewahrt und schliesslich auch im Turbulenzrheometer gemessen. Die Tabelle 1 fasst die Ergebnisse aller Messungen für 25, 55, 70 und 90 °C durch Wiedergabe von $Re_{max}$ und $\alpha_{max}$ zusammen. In der Abbildung sind in der doppeltlogarithmischen Auftragung $\lambda$ gegen Re die Fliesskurven bei 55 °C für 500, 1000 und 2000 ppm Lösungen von $(CF)_8(CH)_4$ TA-Sal in E-Wasser aufgetragen. Wie aus Tabelle 1 zu entnehmen ist, ist der Effekt der Reibungsminderung auch bei Unterschreiten der Löslichkeitstemperatur um ca. 15 °C selbst nach einer Woche noch vorhanden.

Beispiel 8

Unterschiedliche Mengen an NaCl wurden zusammen mit $(CF)_8(CH)_4$ TA-Sal, wie in Beispiel 7 beschrieben, zu wässrigen Lösungen angesetzt, deren Konzentrationen an $(CF)_8(CH)_4$ TA-Sal jeweils 750 ppm (1,15 mmol/l und an NaCl (in mmol/l) wie folgt gewählt wurden:

0,1; 0,5; 1,14; 5; 10; 100; 1000

Die Ergebnisse der Untersuchung auf Reibungsminderung bei 40 °C im Turbulenzrheometer sind in Tabelle 2 zusammengefasst. Wie aus der Tabelle zu entnehmen ist, wirkt sich die NaCl-Zugabe bis 10 mmol/l NaCl nur geringfügig aus; die Höchstkonzentration an NaCl liegt zwischen 100 und 1000 mmol/l. Ebenfalls in Tabelle 2 aufgenommen sind einige Messungen bei 25 °C, d.h. ca. 15 °C unterhalb der Löslichkeitstemperatur.

Beispiel 9

Unterschiedliche Mengen an $Na_2CO_3$ wurden zusammen mit $(CF)_8(CH)_4$ TA-Sal, wie in Beispiel 7 beschrieben, zu wässrigen Lösungen angesetzt, deren Konzentrationen an $(CF)_8(CH)_4$ TA-Sal jeweils 400 ppm (0,62 mmol/l) und an $Na_2CO_3$ (in mmol/l) wie folgt gewählt wurden: 0,1; 0,6; 5. Tabelle 3 fasst die Ergebnisse der Untersuchung auf Reibungsminderung im Turbulenzrheometer bei 40 °C zusammen. Wie aus Tabelle 3 ersichtlich, beeinflusst die Anwesenheit von $Na_2CO_3$ die Wirksamkeit des SB nur geringfügig; die Höchstkonzentration an $Na_2CO_3$ beträgt 5 mmol/l bei pH = 11,2.

Beispiel 10

Unterschiedliche Mengen an $Na_3PO_4$ wurden zusammen mit $(CF)_8(CH)_4$ TA-Sal, wie in Beispiel 7 beschrieben, zu wässrigen Lösungen angesetzt, deren Konzentrationen an $(CF)_8(CH)_4$ TA-Sal jeweils 400 ppm (0,62 mmol/l) und an $Na_3PO_4$ (in mmol/l) wie folgt gewählt wurden: 0,1; 0,6; 5. Tabelle 4 fasst die Ergebnisse der Untersuchung auf Reibungsminderung im Turbulenzrheometer zusammen.

Beispiel 11

Zwei Lösungen von $(CF)_8(CH)_4$ TA-Sal mit einer Konzentration von 400 ppm in E-Wasser wurden mit HCl bzw. NaOH auf die pH-Werte 3 bzw. 10,5 eingestellt, wie in Beispiel 7 beschrieben vorbehandelt und bei 40 °C im Turbulenzrheometer untersucht. Bei pH = 3 ergab sich $Re_{max}$ = 14 900 ($\pm$ 1500) und $\alpha_{max}$ = 63 ($\mp$ 3) und bei pH = 10,5 bei $Re_{max}$ von 16 200 ($\pm$ 1600) und ein $\alpha_{max}$ von 69 ($\pm$ 3). Im Vergleich zur reinen Verbindung in E-Wasser bei pH = 7,4 mit $Re_{max}$ = 15 500 und $\alpha_{max}$ = 65 bewirken die unterschiedlichen pH-Werte nur eine geringfügige, im Rahmen der Messgenauigkeit liegende Änderung der SB-Wirkung.

Beispiel 12

Wie in Beispiel 7 beschrieben, wurden Lösungen in E-Wasser mit unterschiedlichen Konzentrationen an nachfolgend aufgeführten Salicylaten angesetzt und bei unterschiedlichen Temperaturen im Turbulenzrheometer auf Reibungsminderung untersucht.

1. $(C_6F_{13}\, C_4H_8\, N(CH_3)_3)^+\, Sal^-$
   bei 25 °C: $CMC_I = CMC_{II}$ 1110 ppm,
   bei 55 °C: $CMC_I$ = 1600; $CMC_{II}$ = 2560

2. $(C_{10}F_{21}\ C_4H_8\ N(CH_3)_3)^+\ Sal^-$
bei 69,2 °C $CMC_I$ = 85 ppm; $CMC_{II}$ = 581 ppm

3. $(C_8F_{17}\ C_4H_8$ 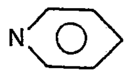 $)^+\ Sal^-$
bei 25 °C $CMC_I$ = $CMC_{II}$ = 480 ppm;
bei 55 °C $CMC_I$ = 720 ppm, $CMC_{II}$ = 2740 ppm.
Sal steht jeweils für das Salicylat-Anion.
Tabelle 5 fasst die Ergebnisse zusammen.

Beispiel 13

Wie in Beispiel 7 beschrieben wurden Lösungen in E-Wasser mit unterschiedlichen Konzentrationen an nachfolgend aufgeführten Salicylaten (Sal) angesetzt und bei unterschiedlichen Temperaturen im Turbulenzrheometer auf Reibungsminderung untersucht.

1. $(C_7F_{15}CF = CHCH_2N(CH_3)_3)^+\ Sal^-$
bei 25 °C: $CMC_I$ = $CMC_{II}$ = 880 ppm
bei 40 °C: $CMC_I$ = $CMC_{II}$ = 1200 ppm
2. $(C_9F_{19}CF = CHCH_2N(CH_3))^+\ Sal^-$, vgl. Beispiel 2
3. $(C_7F_{15}CF = CHCH_2N(CH_3)_2CH_2CH_2OH)^+\ Sal^-$
bei 55 °C: $CMC_I$ = 1390 ppm und $CMC_{II}$ = 1700 ppm (± 250)
bei 25 °C: $CMC_I$ = $CMC_{II}$ = 960 ppm
4. $(C_9F_{19}CF = CHCH_2N(CH_3)_2CH_2CH_2OH)$ Sal
bei 40 °C: $CMC_I$ = 300 ppm, $CMC_{II}$ = 600 ppm (± 200 ppm)

Tabelle 6 fasst sämtliche Ergebnisse zusammen.

Beispiel 14

Unterschiedliche Mengen an NaCl wurden zusammen mit $(C_9F_{19}CF = CHCH_2N(CH_3)_3)$ Sal, wie in Beispiel 7 beschrieben, zu wässrigen Lösungen angesetzt, deren Konzentrationen an $(C_9F_{19}CF{=}CHCH_2N(CH_3)_3)$ Sal jeweils 750 ppm (1 mmol/l) und an NaCl (in mmol/l) wie folgt gewählt wurden: 0,1; 0,5; 1,0; 5; 10; 100; 500. Die Ergebnisse der Untersuchung auf Reibungsminderung im Turbulenzrheometer bei 55 °C sind in Tabelle 7 zusammengefasst. Wie aus der Tabelle zu entnehmen ist, bewirkt die NaCl-Zugabe bis zu einer Konzentration von 10 mmol/l eine deutliche Verbesserung der Wirksamkeit als SB; danach erfolgt eine Abnahme der SB-Wirkung. Die Höchstkonzentration an NaCl ist grösser als 500 mmol/l bzw. 2,9 Gew.-% NaCl.

Beispiel 15

Unterschiedliche Mengen an $CaCl_2$ wurden zusammen mit $(C_9F_{19}CF = CHCH_2N(CH_3)_3)$ Sal, wie in Beispiel 7 beschrieben, zu wässrigen Lösungen angesetzt, deren Konzentrationen an $(C_9F_{19}CF = CHCH_2N(CH_3)_3)$ Sal jeweils 750 ppm (1 mmol/l) und an $CaCl_2$ (in mmol/l) wie folgt gewählt wurden: 0,1; 0,5; 1; 5; 10; 100; 500. Die Ergebnisse der Untersuchung auf Reibungsminderung im Turbulenzrheometer bei 55 °C sind in Tabelle 8 zusammengefasst. Wie die Ergebnisse in Tabelle 8 zeigen, bewirkt die $CaCl_2$-Zugabe bis zu einer Konzentration von 10 mmol/l eine Verbesserung, danach eine Verschlechterung der Wirksamkeit als SB. Die Höchstkonzentration an $CaCl_2$ ist grösser als 500 mmol/l bzw. 5,5 Gew.-%.

Beispiel 16

Unterschiedliche Mengen an $AlCl_3$ wurden zusammen mit $(C_9F_{19}CF = CHCH_2N(CH_3)_3)$ Sal, wie in Beispiel 7 beschrieben, zu wässrigen Lösungen angesetzt, deren Konzentration an $(C_9F_{19}CF = CHCH_2N(CH_3)_3)$ Sal jeweils 750 ppm (1 mmol/l) und an $AlCl_3$ (in mmol/l) wie folgt gewählt wurden: 0,1; 0,5; 1; 5. Die Ergebnisse der Untersuchung auf Reibungsminderung im Turbulenzrheometer bei 55 °C sind in Tabelle 9 zusammengefasst. Wie die Ergebnisse zeigen, bewirkt die Zugabe von $AlCl_3$ keine Verbesserung der SB-Wirkung und ab 1 mmol/l sogar eine völlige Unterdrückung der Reibungsminderung.

Beispiel 17

Wie in Beispiel 7 beschrieben wurden Lösungen in E-Wasser mit unterschiedlichen Konzentrationen der nachfolgend aufgeführten Verbindungen angesetzt und bei unterschiedlichen Temperaturen im Turbulenzrheometer auf Reibungsminderung untersucht.

1. $(C_6F_{13}\ C_4H_8\ N(CH_3)_3)^+\ Naph^-$
2. $(C_8F_{17}\ C_4H_8\ N(CH_3)_3)^+\ Naph^-$
3. $(C_{10}F_{21}\ C_4H_8\ N(CH_3)_3)^+\ Naph^-$
4. $(C_8F_{17}\ C_2H_4$ 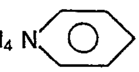 $)^+\ Naph^-$

Es steht Naph für das 3-hydroxy-2-naphthoat Anion:

Tabelle 10 fasst die Ergebnisse zusammen. Die Substanzen 2) und 3) wurden auch, wie in Beispiel 29 beschrieben, in einer anderen Strömungsapparatur bei Temperaturen über 100 °C auf Reibungsminderung geprüft.

Beispiel 18

Wie in Beispiel 7 beschrieben, wurden Lösungen in E-Wasser mit unterschiedlichen Konzentrationen der nachfolgend aufgeführten Verbindungen angesetzt und bei unterschiedlichen Temperaturen im Turbulenzrheometer auf Reibungsminderung untersucht.

1. $(C_9F_{19}CF = CHCH_2N(CH_3)_3)^+\ CF_3COO^-$
bei 55 °C: $CMC_I$ = $CMC_{II}$ = 1940 ppm
2. $(C_9F_{19}CF = CHCH_2N(CH_3)_3)^+\ C_2F_5COO^-$
bei 55 °C: $CMC_I$ = 175 ppm, $CMC_{II}$ = 700 ppm (± 150)
3. $(C_9F_{19}CF = CHCH_2N(CH_3)_3)^+\ C_3F_7COO^-$
bei 70 °C $CMC_I$ = $CMC_{II}$ = 2630 ppm
4. $(C_9F_{19}CF = CHCH_2N(CH_3)_3)^+\ C_4F_5COO^-$
5. $(C_{10}F_{21}\ C_4H_8\ N(CH_3)_3)^+\ C_2F_5COO^-$

Tabelle 11 fasst die Ergebnisse zusammen.

Beispiel 19

Wie in Beispiel 7 beschrieben wurden Lösungen in E-Wasser mit unterschiedlichen Konzentrationen von den nachfolgend aufgeführten Perfluorcarbonaten des n-Hexadecyltrimethylammonium-Kations, $(C_{16}H_{33}N(CH_3)_3)^+$, abgek. $C_{16}\ TA^+$, angesetzt und bei unterschiedlichen Temperaturen ein Turbulenzrheometer auf Reibungsminderung untersucht.

1. $C_{16}\ TA^+\ C_2F_5COO^-$
bei 25 °C: $CMC_I$ = $CMC_{II}$ = 1050 ppm
bei 55 °C: $CMC_I$ = $CMC_{II}$ = 5080 ppm

2. $C_{16}TA^+ \, C_3F_7COO^-$
vgl. Beispiel 5
3. $C_{16}TA^+ \, C_4F_9COO^-$
bei 70 °C: $CMC_I = 240$ ppm, $CMC_{II} = 952$ ppm
4. $C_{16} \, TA^+ \, C_5F_{11}COO^-$
Tabelle 12 fasst die Ergebnisse zusammen.
Die Substanz 3) wurde in einer anderen Strömungsapparatur, vgl. Beispiel 29, auch bei 107 °C auf Reibungsminderung geprüft.

Beispiel 20

Wie in Beispiel 7 beschrieben wurden Lösungen in E-Wasser mit unterschiedlichen Konzentrationen an nachfolgend aufgeführten Verbindungen angesetzt und bei unterschiedlichen Temperaturen im Turbulenzrheometer auf Reibungsminderung untersucht.
1. $(N(CH_3)_4)^+ \, C_8F_{17}SO_3^-$, vgl. Beispiel 6
2. $(N(C_2H_5)_4)^+ \, C_8F_{17}SO_3^-$
bei 25 °C: $CMC_I = CMC_{II} = 650$ ppm
bei 40 °C: $CMC_I = CMC_{II} = 850$ ppm
Tabelle 13 fasst die Ergebnisse zusammen.

Beispiel 21

Wie in Beispiel 7 beschrieben wurden Lösungen in E-Wasser mit unterschiedlichen Konzentrationen von den Jodiden der nachfolgend aufgeführten Verbindungen angesetzt und bei unterschiedlichen Temperaturen im Turbulenzrheometer auf Reibungsminderung untersucht.
1. $(C_8F_{17} \, C_4H_8 \, N(CH_3)_3)^+ \, J^-$
bei 40 °C $CMC_I = CMC_{II} = 450$ ppm ($\pm 50$)

2. $(C_8F_{17} \, C_4H_8$ 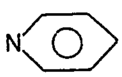 $)^+ \, J^-$

bei 40 °C $CMC_I = CMC_{II} = 380$ ppm ($\pm 50$)
3. $(C_9F_{19}CF = CHCH_2N(CH_3)_3)^+ \, J^-$
4. $R_F \, CF = CHCH_2N(CH_3)_3)^+ \, J^-$
wobei $R_F$ für den Kettenschnitt $C_5F_{11}$ bis $C_{11}F_{23}$ steht.
Tabelle 14 fasst die Ergebnisse zusammen.

Beispiel 22

Wie in Beispiel 7 beschrieben wurden Lösungen in E-Wasser unterschiedlicher n-Fluoralkylpyridinium- und n-Fluoralkyltrimethylammonium-Salicylate angesetzt, indem die entsprechenden n-Fluoralkylpyridinium- und n-Fluoralkyltrimethylammonium-Chloride jeweils 1 : 1 molar mit Na-Salicylat eingewogen wurden, so dass die Lösungen ausser den wirksamen Tensiden äquimolare Mengen an NaCl enthalten. Tabelle 15 fasst die Ergebnisse der Untersuchung auf Reibungsminderung im Turbulenzrheometer zusammen. Die äquimolaren Mengen an NaCl sind in der Tabelle nicht aufgeführt. Die Abkürzung Sal steht für Salicylat.

Beispiel 23

Wie in Beispiel 7 beschrieben wurden Lösungen in E-Wasser von $(C_8F_{17}(CH_2)_4 \, N(CH_3)_3)^+$ mit verschiedenen Gegenionen angesetzt, indem jeweils, wie in Beispiel 22 beschrieben, das Chlorid der n-Fluoralkyltrimethylammoniumverbindung 1 : 1 molar mit dem Na-Salz des entsprechenden Anions eingewogen wurde. Tabelle 16 fasst die Ergebnisse der Untersuchung auf Reibungsminderung im Turbulenzrheometer zusammen. Die äquimolaren Mengen an NaCl sind in der Tabelle nicht aufgeführt. Analog wurde eine Lösung von $(C_6F_{13} \, (CH_2)_4 \, N(CH_3)_3)^+ \, C_3F_7COO^-$ angesetzt, im Turbulenzrheometer untersucht und die Ergebnisse in Tabelle 16 aufgenommen.

Beispiel 24

Wie in Beispiel 7 beschrieben wurden Lösungen in E-Wasser bestehend aus n-Alkyltrimethylammonium-Kationen und Perfluorcarbonat-Anionen angesetzt, indem jeweils die entsprechenden n-Alkyltrimethylammoniumchloride mit den Natriumsalzen der Perfluorcarbonate molar 1 : 1 eingewogen wurden. Tabelle 17 fasst die Ergebnisse der Untersuchung auf Reibungsminderung im Turbulenzrheometer zusammen. Die äquimolaren Mengen an NaCl sind in der Tabelle nicht aufgeführt.

Beispiel 25

Wie in Beispiel 7 beschrieben wurden Lösungen in E-Wasser bestehend aus n-Alkyltrimethylammonium-Kationen, $(C_nH_{2n+1}N(CH_3)_3)^+$, und n-Alkylcarbonat-Anionen, $(C_xH_{2x+1}COO)^-$, angesetzt, indem jeweils die entsprechenden n-Alkyltrimethylammoniumchloride mit den Natriumsalzen der n-Alkylcarbonsäuren molar 1 : 1 eingewogen wurden. Tabelle 18 fasst die Ergebnisse der Untersuchung auf Reibungsminderung zusammen. Die äquimolaren Mengen an NaCl sind in der Tabelle nicht aufgeführt.

Beispiel 26

Von den Verbindungen $(C_nH_{2n+1}NH_3)^+ \, CF_3COO^-$ wurden Lösungen in E-Wasser bei 60 °C angesetzt, indem jeweils die entsprechenden primären Amine $C_nH_{2n+1}NH_2$ mit Perfluoressigsäure molar im Verhältnis 1 : 1 eingewogen wurden. Nach einer Aufbewahrungszeit von ca. 7 Tagen bei 60 °C wurden die Lösungen im Turbulenzrheometer auf Reibungsminderung untersucht. Tabelle 19 fasst die Ergebnisse zusammen.

Zur Erzielung einer optimalen Wirksamkeit als SB sollten die Verbindungen vom Typ $(C_nH_{2n+1} \, NH_3)^+ \, A^-$ nicht über 70 °C erwärmt werden.

Beispiel 27

Wie in Beispiel 7 beschrieben wurde eine Lösung in E-Wasser mit einer Konzentration von 1000 Gew.-ppm der nachfolgend aufgeführten Verbindungen angesetzt und bei unterschiedlichen Temperaturen im Turbulenzrheometer auf Reibungsminderung untersucht.
1. $(C_8F_{17}SO_2 \, N(C_2H_5) \, (CH_2)_3 \, N(CH_3)_3)^+ \, J^-$
(abgek.: $SO_2TA^+ \, J^-$)

Analog angesetzt und untersucht wurden Lösungen der Salicylate (Sal) der folgenden Verbindungen:
3. $(C_8F_{17}SO_2N(C_2H_5)(CH_2)_3 \, N(CH_3)_3)^+Sal^-$
(abgek. $SO_2TA^+$–$Sal^-$)
4. $(R_FCH_2CH_2SCH_2CH_2N(CH_3)_3)^+Sal^-$
(abgek.: $STA^+$–$Sal^-$)

$R_F$ steht für die Kettenmischung $C_6F_{13}$ bis $C_{12}F_{25}$. Tabelle 20 fasst die Ergebnisse zusammen.

Beispiel 28

Zur Untersuchung der Reibungsminderung in einem Dauerversuch bei 70 °C wurde eine geschlossene Strömungsapparatur benutzt, bestehend aus einem 30 l Vorratsgefäss, einer Kreiselpumpe (Typ: CPK 50–250 der Firma KSB mit mechanischem Regelgetriebe), einem induktiven Durchflussmesser und einer Rohrleitung von 2 × 10 m Länge mit einem Innendurchmesser von 29,75 mm. Die Bestimmung des Druckabfalls $\Delta P$ erfolgt nach einer gehörigen Einlaufstrecke über eine Messstrecke von 2 m. Zur Thermostatisierung dient ein Tauchsieder, der die Flüssigkeit im Vorratsbehälter elektrisch aufheizt. Während des Dauerversuches wird die Flüssigkeit ständig am Boden des Vorratsgefässes abgepumpt und über die Rohrleitung dem Vorratsbehälter wieder zugeführt. Für Wasser bei 70 °C lässt sich die Förderleistung der Pumpe mit Hilfe des mechanischen Regelgetriebes von 3,25 bis 14,3 $m^3/h$ variieren; das entspricht bei einem Rohrdurchmesser von 29,75 mm den Strömungsgeschwindigkeiten von 1,3 bis 5,7 m/s bzw. den Reynoldszahlen von 94 000 bis 411 000.

Eine 1000 ppm Lösung von $(C_8F_{17}(CH_2)_4N(CH_3)_3)$ Sal (Sal steht für das Salicylsäure-Anion), wurde in dieser Apparatur auf ihre Zeitbeständigkeit als Strömungsbeschleuniger bei 70 °C untersucht. Das Ansetzen der Lösung erfolgte hier derart, dass die Apparatur (Fassungsvermögen ca. 40 l) bis auf einen geringen Rest mit Wasser gefüllt wurde, dann 40 g des pulverförmigen Strömungsbeschleunigers zugegeben und anschliessend die Apparatur mit dem restlichen Wasser aufgefüllt wurde. Nach einer Einlaufzeit von 3 Tagen bei 70 °C ergab sich aus der Aufnahme der Fliesskurve (vgl. die beigefügte Zeichnung) ein $Re_{max}$ von 332 000 ($\pm$30 000) und $\alpha_{max}$ = 76% $\pm$4.

Der Dauerversuch wurde daraufhin bei einem Durchfluss von 10,26 $m^3/h$, entsprechend einer Strömungsgeschwindigkeit von 4,1 m/s bzw. Re = 295 000 über einen Zeitraum von 19 Tagen durchgeführt. Unter den angegebenen Bedingungen passiert das gleiche Flüssigkeitsvolumen ca. 4,3 mal pro Minute die Kreiselpumpe. Nach 19tägiger Dauerbeanspruchung ergab die erneute Aufnahme der Fliesskurve ein $Re_{max}$ = 329 000 ($\pm$30 000) und ein $\alpha_{max}$ = 77% $\pm$4, so dass ein Nachlassen der Wirksamkeit durch Abbau nicht feststellbar war.

Beispiel 29

Zur Untersuchung der Reibungsminderung im Temperaturbereich von 70 bis 135 °C wurde eine geschlossene Strömungsapparatur benutzt, in der ein Flüssigkeitsvolumen von ca. 1,5 l im Kreislauf umgepumpt wird. Mit zwei parallel geschalteten Netzsch-Mohno-Pumpen Typ 3 NU 06 mit einer Förderleistung von 10 bis 150 l/h und Typ 2 Ne 15A mit einer Förderleistung von 80 bis 1500 l/h, lassen sich im Messrohr mit dem Durchmesser 6 mm Strömungsgeschwindigkeiten von insgesamt 0,2 bis 14 m/s einstellen. Die Bestimmung der Strömungsgeschwindigkeit erfolgt mit einem induktiven Durchflussmesser; der Druckabfall $\Delta P$ wird nach einer Einlaufstrecke von 1,83 m über eine Länge von 1 m mit Hilfe von Ziegler Differenzdruckmessdosen (Typ D 248) bestimmt. Messrohr und Rücklaufrohr sind parallel angeordnet und zwecks schnellerer Entlüftung senkrecht aufgestellt. Während ein kombinierter Druckausgleichs- und Entlüftungsbehälter an der oberen Querverbindung der beiden parallel laufenden Rohre angebracht ist, werden am unteren Ende beide Rohrleitungen über die parallel geschalteten Pumpen verbunden. Die gesamte Rohrleitung, der Ausgleichsbehälter, sowie Teile der Pumpen sind ummantelt und werden von einem Thermostaten mit Badflüssigkeit temperiert. Die Messwerterfassung und Auswertung der Messungen erfolgt über einen HP 1000 Rechner.

Lösungen der nachfolgend aufgeführten Verbindungen in E-Wasser mit unterschiedlichen Konzentrationen wurden in dieser Apparatur bei unterschiedlichen Temperaturen untersucht.

1. $(C_8F_{19}\ (CH_2)_4\ N(CH_3)_3)^+$ $Naph^-$
2. $(C_{10}F_{21}\ (CH_2)_4\ N(CH_3)_3)^+$ $Sal^-$
3. $(C_{10}F_{21}(CH_2)_4\ N(CH_3)_3)^+$ $Naph^-$
4. $(C_{16}H_{33}\ N(CH_3)_3)^+$ $C_4F_9\ COO^-$

(Es steht $Sal^-$ für das Salicylat-Anion und $Naph^-$ für das Anion der 3-Hydroxy-2-naphthoesäure.)

Nach dem Ansetzen der Lösungen, wie in Beispiel 7 beschrieben, erfolgte die weitere Temperierung der Lösungen vor einer neuen Messung bei einer anderen Temperatur in der Strömungsapparatur selbst, indem die Lösungen jeweils für einige Stunden (bzw. über Nacht) bei der neuen Messtemperatur im Kreislauf umgepumpt wurden. Die Temperierung der Proben für einige Stunden ist bei Temperaturen über 90 °C ausreichend, da die Gleichgewichtseinstellung des Systems (bzw. die Zeit, die notwendig ist, um den konstanten Endwert für die Reibungsminderung zu erhalten) in diesem Temperaturbereich sehr kurz ist, wie in einzelnen Testmessungen ermittelt wurde.

Tabelle 21 fasst die Ergebnisse wiederum in Form von $Re_{max}$ und $\alpha_{max}$ zusammen.

Tabelle 1

$(CF)_8\ (CH)_4$ TA-Sal

| T/°C | Konzentration/ppm | $Re_{max}$ | $\alpha_{max}$ |
|---|---|---|---|
| 25 | 200 | 7200 $\pm$ 700 | 60 $\pm$ 3 |
| 25 | 500 | 12800 $\pm$ 1300 | 72 $\pm$ 3 |
| 25 | 750 | 17300 $\pm$ 1700 | 74 $\pm$ 3 |
| 25 | 1000 | 16800 $\pm$ 1700 | 75 $\pm$ 3 |
| 55 | 500 | 18000 $\pm$ 1800 | 69 $\pm$ 3 |
| 55 | 750 | 22400 $\pm$ 2200 | 71 $\pm$ 3 |
| 55 | 1000 | 27200 $\pm$ 2700 | 74 $\pm$ 3 |
| 55 | 1500 | 41700 $\pm$ 4200 | 75 $\pm$ 3 |
| 55 | 2000 | 48700 $\pm$ 4900 | 75 $\pm$ 3 |

Tabelle 1 (Fortsetzung)

| T/°C | Konzentration/ppm | $Re_{max}$ | $\alpha_{max}$ |
|---|---|---|---|
| 70 | 500 | 9400 ± 940 | 70 ± 3 |
| 70 | 750 | 15600 ± 1600 | 72 ± 3 |
| 70 | 1000 | 27300 ± 2700 | 70 ± 3 |
| 70 | 1500 | 38000 ± 9800 | 69 ± 3 |
| 70 | 2000 | 41300 ± 4100 | 74 ± 3 |
| 90 | 2000 | 23200 ± 2360 | 31 ±3 |

Tabelle 2
$(CF)_8$ $(CH)_4$ TA-Sal-Konz: 750 ppm (1,15 mmol/l)

| T/°C | NaCl-Konzentration/mmol/L | $Re_{max}$ | $\alpha_{max}$ |
|---|---|---|---|
| 40 | 0 | 21100 ± 2100 | 76 ± 4 |
| 40 | 0,1 | 22200 ± 2200 | 70 ± 3 |
| 40 | 0,5 | 20200 ± 2000 | 71 ± 3 |
| 40 | 0,14 | 23400 ± 2300 | 71 ± 3 |
| 40 | 5 | 23700 ± 2400 | 71 ± 3 |
| 40 | 10 | 18700 ± 1900 | 71 ± 3 |
| 40 | 100 | 16600 ± 1700 | 68 ± 3 |
| 25 | 1,14 | 13900 ± 1400 | 68 ± 3 |
| 25 | 10 | 16200 ± 1600 | 61 ± 3 |
| 25 | 100 | 8300 ± 800 | 62 ± 3 |
| 25 | 1000 | 6000 ± 600 | 57 ± 3 |

Tabelle 3
$(CF)_8$ $(CH)_4$ TA-Sal-Konz: 400 ppm (0,62 mmol/l)
Messtemperatur 40 °C

| $Na_2CO_3$-Konz./mmol/l | pH-Wert | $Re_{max}$ | $\alpha_{max}$ |
|---|---|---|---|
| 0,1 | 8,6 | 15900 ± 1600 | 67 ± 3 |
| 0,6 | 10,3 | 15700 ± 1600 | 63 ± 3 |
| 5 | 11,2 | 12600 ± 1300 | 66 ± 3 |

Tabelle 4
$(CF)_8$ $(CH)_4$ TA-Sal-Konz: 400 ppm (0,62 mmol/l)
Messtemperatur 40 °C

| $Na_3PO_4$-Konz./mmol/l | pH-Wert | $Re_{max}$ | $\alpha_{max}$ |
|---|---|---|---|
| 0 | 7,4 | 15500 ± 1600 | 65 ± 3 |
| 0,1 | 8,3 | 15700 ± 1600 | 65 ± 3 |
| 0,6 | 9,8 | 15400 ± 1500 | 68 ± 3 |
| 5 | 10,7 | 12700 ± 1300 | 66 ± 3 |

Tabelle 5

| Substanz | Konz./ppm | T/°C | $Re_{max}$ | $\alpha_{max}$ |
|---|---|---|---|---|
| $(C_6F_{13}C_4H_8N(CH_3)_3)^+$ $Sal^-$ | 2000 | 25 | 24400 ± 2400 | 74 ± 4 |
| " | 3000 | 25 | 35700 ± 3600 | 75 ± 4 |
| " | 3000 | 40 | 33800 ± 3400 | 78 ± 4 |
| " | 3000 | 55 | 48900 ± 5000 | 26 ± 2 |
| $(C_{10}F_{21}C_4H_8N(CH_3)_3)^+$ $Sal^-$ | 1000 | 70 | 32500 ± 3300 | 76 ± 4 |
| " | 1000 | 90 | 32600 ± 3300 | 71 ± 3 |
| $(C_8F_{17}C_2H_4$ 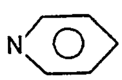 $)^+$ $Sal^-$ | 750 | 25 | 11700 ± 1200 | 62 ± 3 |

Tabelle 6

| Substanz | Konz./ppm | T/°C | $Re_{max}$ | $\alpha_{max}$ |
|---|---|---|---|---|
| $(C_7F_{15}$ $CF=CHCH_2N(CH_3)_3)^+$ $Sal^-$ | 1500 | 25 | 18000 ± 1800 | 67 ± 3 |
| " | 1500 | 40 | 10400 ± 1000 | 65 ± 3 |
| $(C_9F_{19}$ $CF=CHCH_2N(CH_3)_3)^+$ $Sal^-$ | 200 | 25 | 6600 ± 700 | 62 ± 3 |
| " | 1000 | 25 | 7000 ± 700 | 57 ± 3 |
| " | 1000 | 55 | 10600 ± 1100 | 58 ± 3 |
| " | 1000 | 70 | 8400 ± 800 | 67 ± 3 |
| $(C_7F_{15}CF=CHCH_2N(CH_3)_2CH_2CH_2OH)^+$ $Sal^-$ | 1600 | 25 | 21100 ± 2100 | 66 ± 3 |
| " | 2000 | 55 | 26000 ± 2600 | 68 ± 3 |
| $(C_9F_{19}CF=CHCH_2N(CH_3)_2CH_2CH_2OH)^+$ $Sal^-$ | 1000 | 40 | 12300 ± 1200 | 70 ± 4 |

Tabelle 7
($(C_9F_{19}CF = CHCH_2N(CH_3)_3)^+$ $Sal^-$-Konz.: 750 ppm (1 mmol/l)
Messtemperatur 55 °C

| NaCl-Konz./ mmol/l | $Re_{max}$ | $\alpha_{max}$ |
|---|---|---|
| 0,1 | 10800 ± 1100 | 57 ± 3 |
| 0,5 | 13200 ± 1300 | 64 ± 3 |
| 1 | 15200 ± 1500 | 66 ± 3 |
| 5 | 21600 ± 2200 | 67 ± 3 |
| 10 | 21300 ± 2100 | 69 ± 3 |
| 100 | 12000 ± 1200 | 66 ± 3 |
| 500 | 10600 ± 1100 | 71 ± 4 |

Tabelle 8
($(C_9F_{19}CF=CHCH_2N(CH_3)_3)^+$ $Sal^-$-Konz.: 750 ppm (1 mmol/l)
Messtemperatur 55 °C

| $CaCl_2$-Konz. mmol/l | $Re_{max}$ | $\alpha_{max}$ |
|---|---|---|
| 0,1 | 10500 ± 1000 | 63 ± 3 |
| 0,5 | 12700 ± 1300 | 59 ± 3 |
| 1 | 14300 ± 1400 | 65 ± 3 |
| 5 | 19400 ± 1900 | 70 ± 4 |
| 10 | 18300 ± 1800 | 72 ± 4 |
| 100 | 14600 ± 1500 | 69 ± 3 |
| 500 | 10800 ± 1000 | 62 ± 3 |

Tabelle 9
$(C_9F_{19}CF=CHCH_2N(CH_3)_3)$ Sal-Konz.: 750 ppm
Messtemperatur: 55 °C

| $AlCl_3$-Konz./ mmol/l | $Re_{max}$ | $\alpha_{max}$ |
|---|---|---|
| 0,1 | 10000 ± 1000 | 56 ± 3 |
| 0,5 | 11900 ± 1200 | 64 ± 3 |

Tabelle 10

| Substanz | Konz./ppm | T/°C | $Re_{max}$ | $\alpha_{max}$ |
|---|---|---|---|---|
| $(C_6F_{13}C_4H_8N(CH_3)_3)^+$ $Naph^-$ | 1200 | 70 | 30900 ± 3100 | 59 ± 3 |
| $(C_8F_{17}C_4H_8N(CH_3)_3)^+$ $Naph^-$ | 1500 | 70 | 26000 ± 2700 | 75 ± 4 |
| " | 1500 | 90 | 57900 ± 5800 | 74 ± 4 |
| $(C_{10}F_{21}C_4H_8N(CH_3)_3)^+$ $Naph^-$ | 750 | 70 | 18900 ± 1900 | 76 ± 4 |
| " | 750 | 90 | 32300 ± 3500 | 76 ± 4 |
| $(C_8F_{17}C_2H_4$ N 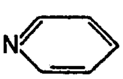 $)^+$ $Naph^-$ | 1000 | 60 | 10700 ± 1100 | 61 ± 3 |
| " | 1000 | 70 | 19000 ± 1900 | 46 ± 2 |

Tabelle 11

| Substanz | Konz./ppm | T/°C | $Re_{max}$ | $\alpha_{max}$ |
|---|---|---|---|---|
| $(C_9F_{19}CF=CHCH_2N(CH_3)_3)^+$ $CF_3COO^-$ | 3500 | 50 | 9100 ± 900 | 57 ± 3 |
| $(C_9F_{19}CF=CHCH_2N(CH_3)_3)^+$ $C_2F_5COO^-$ | 2000 | 50 | 18700 ± 1900 | 73 ± 4 |
| $(C_9F_{19}CF=CHCH_2N(CH_3)_3)^+$ $C_3F_7COO^-$ | 3500 | 70 | 9400 ± 900 | 62 ± 3 |
| $(C_9F_{19}CF=CHCH_2N(CH_3)_3)^+$ $C_4F_5COO^-$ | 2860 | 90 | 9700 ± 1000 | 34 ± 2 |
| $(C_{10}F_{21}C_4H_8N(CH_3)_3)^+$ $C_2F_5COO^-$ | 1500 | 70 | 16300 ± 1600 | 68 ± 3 |
| " | 1500 | 90 | 5300 ± 500 | 57 ± 3 |

Tabelle 12

| Substanz | Konz./ppm | T/°C | $Re_{max}$ | $\alpha_{max}$ |
|---|---|---|---|---|
| $C_{16}TA^+\ C_2F_5COO^-$ | 1500 | 25 | 4500 ± 500 | 44 ± 3 |
| $C_{16}TA^+\ C_4F_9COO^-$ | 2000 | 70 | 12600 ± 1300 | 62 ± 3 |
| " | 2000 | 90 | 34400 ± 3400 | 76 ± 4 |
| $C_{16}TA^+\ C_5F_{11}COO^-$ | 3500 | 90 | 17800 ± 1800 | 52 ± 3 |
| $C_{16}TA^+\ C_3F_7COO^-$ | 200 | 25 | 6800 ± 700 | 55 ± 3 |
| " | 300 | 25 | 7600 ± 800 | 58 ± 3 |
| " | 400 | 25 | 8100 ± 800 | 59 ± 3 |
| " | 500 | 25 | 9100 ± 900 | 61 ± 3 |
| " | 750 | 25 | 11700 ± 1100 | 65 ± 4 |
| " | 1000 | 25 | 14600 ± 1500 | 68 ± 4 |
| $C_{16}TA^+\ C_3F_7COO^-$ | 200 | 50 | 5600 ± 600 | 55 ± 3 |
| " | 300 | 50 | 7000 ± 800 | 56 ± 3 |
| " | 400 | 50 | 8900 ± 900 | 59 ± 3 |
| " | 500 | 50 | 12500 ± 1300 | 60 ± 3 |
| " | 750 | 50 | 18600 ± 1900 | 68 ± 3 |
| " | 1000 | 50 | 23500 ± 2400 | 71 ± 3 |
| $C_{16}TA^+\ C_3F_7COO^-$ | 500 | 70 | 9000 ± 900 | 17 ± 2 |
| " | 750 | 70 | 12700 ± 1300 | 46 ± 2 |
| " | 1000 | 70 | 17000 ± 1700 | 71 ± 3 |

Tabelle 13

| Substanz | Konz./ppm | T/°C | $Re_{max}$ | $\alpha_{max}$ |
|---|---|---|---|---|
| $(N(CH_3)_4)^+\ C_8F_{17}SO_3^-$ | 2000 | 40 | 15500 ± 1600 | 60 ± 3 |
| $(N(C_2H_5)_4)^+\ C_8F_{17}SO_3^-$ | 1000 | 22 | 13000 ± 1300 | 69 ± 3 |
| " | 2000 | 22 | 24100 ± 2400 | 69 ± 3 |
| " | 1000 | 40 | 11500 ± 1200 | 50 ± 3 |
| " | 2000 | 40 | 20100 ± 2000 | 70 ± 3 |

Tabelle 14

| Substanz | Konz./ppm | T/°C | $Re_{max}$ | $\alpha_{max}$ |
|---|---|---|---|---|
| $(C_8F_{17}\ C_4H_8$ N 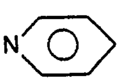 $)^+\ J^-$ | 1000 | 40 | 9600 ± 1000 | 52 ± 3 |
| " | 2000 | 40 | 13200 ± 1300 | 56 ± 3 |
| $(C_8F_{17}\ C_4H_8\ N(CH_3)_3)^+\ J^-$ | 800 | 40 | 12000 ± 1200 | 55 ± 3 |
| $(C_9F_{19}CF{=}CHCH_2N(CH_3)_3)^+\ J^-$ | 1000 | 55 | 7400 ± 700 | 53 ± 3 |
| $(R_F\ CF{=}CHCH_2N(CH_3)_3)^+\ J^-$ | 1000 | 40 | 4200 ± 400 | 52 ± 3 |

Tabelle 15

| Substanz* | Konz./ppm | T/°C | $Re_{max}$ | $\alpha_{max}$ |
|---|---|---|---|---|
| $(C_4F_9(CH_2)_4N(CH_3)_3)^+$ $Sal^-$ | 4000 | 25 | 4500 ± 400 | 55 ± 3 |
| $(C_8F_{19}(CH_2)_4N(CH_3)_3)^+$ $Sal^-$ | 1000 | 40 | 23600 ± 2400 | 60 ± 3 |
| $(C_6F_{19}(CH_2)_4N$ 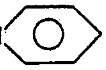 $)^+$ $Sal^-$ | 3000 | 40 | 24500 ± 2400 | 48 ± 2 |
| $(C_8F_{19}(CH_2)_4$ N 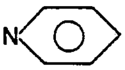 $)^+$ $Sal^-$ | 300 | 53 | 13700 ± 1400 | 61 ± 3 |
| " | 500 | 53 | 12700 ± 1300 | 63 ± 3 |
| " | 1000 | 53 | 26300 ± 2600 | 69 ± 3 |
| " | 1500 | 53 | 32300 ± 3200 | 73 ± 4 |
| " | 1000 | 70 | 14300 ± 1400 | 71 ± 4 |
| " | 1500 | 70 | 25200 ± 2500 | 71 ± 4 |
| $(C_{10}F_{21}(CH_2)_4$ N 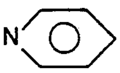 $)^+$ $Sal^-$ | 300 | 60 | 9400 ± 900 | 64 ± 3 |
| " | 500 | 60 | 15100 ± 1500 | 69 ± 3 |
| " | 1000 | 60 | 25800 ± 2600 | 74 ± 4 |
| " | 1500 | 60 | 36100 ± 3600 | 74 ± 4 |

* Die Lösungen enthalten jeweils equimolare Mengen an NaCl

Tabelle 16

| Substanz* | | Konz./ppm | T/°C | $Re_{max}$ | $\alpha_{max}$ |
|---|---|---|---|---|---|
| $(CF)_8TA^+$ | $CCl_3COO^-$ | 2000 | 25 | 13800 ± 1400 | 62 ± 3 |
| " | $CCl_3COO^-$ | 2000 | 55 | 13600 ± 1400 | 61 ± 3 |
| " | $C_6H_{19}SO_3^-$ | 2000 | 40 | 24200 ± 2400 | 71 ± 4 |
| " | $C_7H_{15}SO_3^-$ | 2000 | 40 | 9200 ± 900 | 59 ± 3 |
| " | $C_6H_{13}COO^-$ | 2000 | 25 | 12800 ± 1300 | 56 ± 3 |
| " | $C_7H_{15}COO^-$ | 2000 | 25 | 19500 ± 1900 | 70 ± 3 |
| " | $C_7H_{15}COO^-$ | 2000 | 55 | 27300 ± 2700 | 71 ± 4 |
| " | $C_8H_{17}COO^-$ | 3000 | 40 | 7700 ± 800 | 59 ± 3 |
| " | $SCN^-$ | 2000 | 50 | 23400 ± 2300 | 68 ± 3 |
| $(CF)_8TA^+$ | $CF_3COO^-$ | 2000 | 40 | 17200 ± 1700 | 62 ± 3 |
| " | $C_3F_7COO^-$ | 300 | 40 | 6500 ± 700 | 49 ± 3 |
| $(CF)_6TA^+$ | $C_3F_7COO^-$ | 2000 | 25 | 12000 ± 1200 | 67 ± 3 |

$(CF)_8TA^+$ steht für $(C_8F_{17}(CH_2)_4N(CH_3)_3)^+$
$(CF)_6TA^+$ steht für $(C_6F_{13}(CH_2)_4N(CH_3)_3)^+$

* Die Lösungen enthalten jeweils equimolare Mengen an NaCl

Tabelle 17

| Substanz* | | Konz./ppm | T/°C | $Re_{max}$ | $\alpha_{max}$ |
|---|---|---|---|---|---|
| $C_{14}TA^+$ | $C_3F_7COO^-$ | 500 | 25 | 9600 ± 1000 | 61 ± 3 |
| " | " | 2000 | 25 | 27900 ± 2800 | 72 ± 4 |
| " | " | 2000 | 55 | 30900 ± 3100 | 59 ± 3 |
| $C_{18}TA^+$ | $C_3F_7COO^-$ | 500 | 50 | 11300 ± 1100 | 64 ± 3 |
| " | " | 2000 | 50 | 51700 ± 5200 | 77 ± 4 |
| " | " | 2000 | 88 | 30900 ± 3100 | 70 ± 4 |

Tabelle 17 (Fortsetzung)

| Substanz* | | Konz./ppm | T/°C | $Re_{max}$ | $\alpha_{max}$ |
|---|---|---|---|---|---|
| $C_{10}TA^+$ | $C_6F_{13}COO^-$ | 2000 | 40 | 14700 ± 1500 | 56 ± 3 |
| $C_8TA^+$ | $C_6F_{13}COO^-$ | 2000 | 25 | 4400 ± 400 | 30 ± 2 |
| " | " | 2000 | 40 | 4800 ± 500 | 58 ± 3 |
| $C_6TA^+$ | $C_7F_{15}COO^-$ | 3000 | 40 | 4200 ± 400 | 34 ± 2 |
| $C_4TA^+$ | $C_8F_{17}COO^-$ | 2000 | 40 | 21200 ± 2100 | 72 ± 4 |
| $C_4TA^+$ | $C_9F_{19}COO^-$ | 2000 | 50 | 34600 ± 3500 | 69 ± 3 |
| " | " | | 60 | 29300 ± 2900 | 71 ± 4 |
| $C_1TA^+$ | $C_9F_{19}COO^-$ | 2000 | 40 | 8800 ± 900 | 47 ± 2 |

$C_nTA^+$ steht jeweils für $(C_nH_{2n+1}N(CH_3)_3)^+$

* Die Lösungen enthalten jeweils equimolare Mengen an NaCl

Tabelle 18

| Substanz* | | Konz./ppm | T/°C | $Re_{max}$ | $\alpha_{max}$ |
|---|---|---|---|---|---|
| $C_{14}TA^+$ | $C_9H_{19}COO^-$ | 1000 | 40 | 15500 ± 1600 | 69 ± 3 |
| " | " | 2000 | 40 | 41400 ± 4000 | 75 ± 4 |
| " | $C_{10}H_{21}COO^-$ | 1500 | 50 | 12300 ± 1200 | 53 ± 2 |
| $C_{18}TA^+$ | $C_7H_{15}COO^-$ | 5000 | 50 | 4300 ± 400 | 40 ± 2 |
| " | " | 2000 | 60 | 4100 ± 400 | 45 ± 2 |
| " | $C_9H_{19}COO^-$ | 250 | 60 | 7200 ± 70 | 61 ± 3 |
| " | " | 1000 | 60 | 25600 ± 2500 | 73 ± 4 |
| $C_{20/22}TA^+$ | $C_7H_{15}COO^-$ | 5000 | 50 | 55300 ± 5500 | 73 ± 4 |
| " | $C_8H_{17}COO^-$ | 2000 | 55 | 40700 ± 4100 | 74 ± 4 |
| " | $C_9H_{19}COO^-$ | 1000 | 50 | 28700 ± 2900 | 70 ± 4 |
| " | " | 2000 | 50 | 36700 ± 3800 | 76 ± 4 |
| " | " | 1000 | 88 | 78400 ± 7800 | 79 ± 4 |

$C_nTA^+$ steht jeweils für $(C_nH_{2n+1}N(CH_3)_3)^+$

* Die Lösungen enthalten jeweils equimolare Mengen an NaCl

Tabelle 19

| Substanz | | Konz./ppm | T/°C | $Re_{max}$ | $\alpha_{max}$ |
|---|---|---|---|---|---|
| $(C_{14}H_{29}NH_3)^+$ | $CF_3COO^-$ | 1500 | 60 | 35600 ± 3600 | 72 ± 4 |
| $(C_{16}H_{33}NH_3)^+$ | $CF_3COO^-$ | 500 | 60 | 8300 ± 800 | 36 ± 3 |
| " | " | 1000 | 60 | 8100 ± 800 | 50 ± 3 |
| " | " | 1500 | 60 | 15400 ± 1500 | 52 ± 3 |
| $(C_{18}H_{37}NH_3)^+$ | $CF_3COO^-$ | 1800 | 60 | 4200 ± 400 | 36 ± 3 |

Tabelle 20

| Sustanz | Konz./ppm | T/°C | $Re_{max}$ | $\alpha_{max}$ |
|---|---|---|---|---|
| $SO_2TA$-J | 1000 | 40 | 4800 ± 500 | 37 ± 2 |
| " | 1000 | 55 | 5500 ± 600 | 37 ± 3 |
| $SO_2TA$-Sal | 1000 | 40 | 8500 ± 900 | 53 ± 2 |
| " | 1000 | 55 | 10000 ± 1000 | 55 ± 2 |
| STA-Sal | 2000 | 23 | 4300 ± 400 | 47 ± 2 |
| " | 3000 | 22 | 4900 ± 1000 | 55 ± 2 |

Tabelle 21

| Substanz | Konz./ppm | T/°C | $Re_{max}$ | $\alpha_{max}$ |
|---|---|---|---|---|
| $(C_8F_{19}(CH_2)_4N(CH_3)_3)^+$ $Naph^-$ | 1500 | 62 | 17100 ± 1700 | 63 ± 3 |
| " | 1500 | 71 | 23400 ± 2300 | 68 ± 3 |
| " | 1500 | 90 | 29600 ± 3000 | 74 ± 3 |
| " | 2000 | 97 | 20100 ± 20000 | 83 ± 5 |
| " | 2000 | 113 | 205000 ± 20000 | 82 ± 5 |
| " | 2000 | 129 | 156000 ± 16000 | 72 ± 4 |
| $(C_{10}F_{21}(CH_2)_4N(CH_3)_3)^+$ $Sal^-$ | 1000 | 96 | 32000 ± 3000 | 84 ± 5 |
| " | 1000 | 106 | 43200 ± 4000 | 85 ± 5 |
| " | 1000 | 116 | 31000 ± 3000 | 78 ± 5 |
| $C_{10}F_{21}(CH_2)_4N(CH_3)_3)^+$ $Naph^-$ | 1500 | 119 | 180000 ± 18000 | 82 ± 5 |
| " | 1500 | 126 | 175000 ± 17500 | 82 ± 5 |
| " | 1500 | 134 | 162000 ± 16200 | 76 ± 4 |
| $(C_{16}H_{33}N(CH_3)_3)^+$ $C_4F_9COO^-$ | 2000 | 97 | 33900 ± 3400 | 51 ± 3 |
| " | 2000 | 107 | 13800 ± 1400 | 50 ± 3 |

**Patentansprüche**

1. Quaternäre Ammoniumverbindungen der Formel

$$R\text{–}K^+A^-$$

wobei R n-Fluoralkyl, n-Fluoralkenyl, n-Alkyl, n-Alkenyl oder eine Gruppe der Formeln

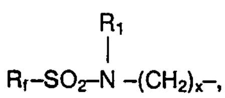,
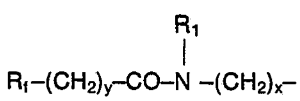

oder

$$R_f\text{–}(CH_2)_yB(CH_2)_x\text{–}$$

$R_f$ n-Fluoralkyl oder n-Fluoralkenyl, $R_1$ Wasserstoff, Methyl oder Ethyl, X eine ganze Zahl von 2 bis 6, y 0, 1 oder 2, B ein Sauerstoff- oder Schwefelatom,

$K^+$ ein Kation der Formeln

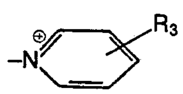

oder

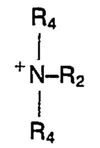

$R_2$ Wasserstoff, $C_1$–$C_4$-Hydroxyalkyl, $C_1$–$C_5$-Alkyl, Benzyl oder Phenyl, $R_3$ Wasserstoff, Methyl oder Ethyl, $R_4$ jeweils gleich oder verschieden sein können, Wasserstoff oder $C_1$–$C_3$-Alkyl und $A^-$ für den Fall der fluorierten Gruppen unter der Bedeutung von R ein n-Alkyl-, n-Alkenyl- oder n-Fluoralkylsulfonat-Anion, ein n-Alkyl-, n-Alkenyl- oder n-Fluoralkenylcarboxylat-Anion, ein Benzoat-, Phenylsulfonat-, Naphthoat-, Rhodanid-Anion oder ein Anion der Formel $C_n\,Cl_{2n+1}\,COO^-$ mit n = 1, 2 oder 3 und $A^-$ für den Fall der nichtfluorierten Gruppe unter der Bedeutung von R ein n-Fluoralkyl- oder n-Fluoralkenylcarboxylat-Anion oder ein n-Fluoralkylsulfonat- oder n-Fluoralkenylsulfonat-Anion bedeuten, wobei die Verbindungen ausgenommen sind für die Fälle R = Fluoralkyl, Fluoralkenyl und $K^+$ Trialkylammonium und $A^-$ = Alkylsulfonat, Alkenylsulfonat, Phenylsulfonat, Acetat; R = Alkyl und $K^+$ = $N^+H_3$ und $A^-$ = Fluoralkylcarboxylat und R–$K^+$ = ($C_1$–$C_2$)-Trialkylammonium, $A^-$ = Fluoralkylsulfonat und Fluoralkylcarboxylat, ebenso die Salze der Formel

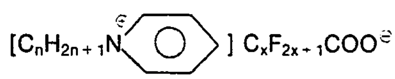

für n = 12–14
und x = 1–3.

2. Quartäre Ammoniumverbindungen der Formel R–$K^+A^-$ wobei R $C_8$–$C_{14}$-n-Fluoralkyl, $C_8$–$C_{14}$-n-Fluoralkenyl, $C_1$–$C_{24}$-n-Alkyl oder $C_1$–$C_{24}$-n-Alkenyl, oder eine Gruppe der Formeln

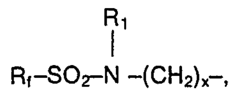,
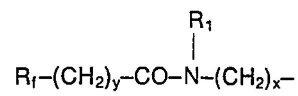

oder

$$R_f\text{–}(CH_2)_yB(CH_2)_x\text{–}$$

$R_f$ $C_8$–$C_{14}$-n-Fluoralkyl oder $C_8$–$C_{14}$-n-Fluoralkenyl, $R_1$ Methyl oder Ethyl, X eine ganze Zahl von 2 bis 6, y 0, 1 oder 2, B ein Sauerstoff- oder Schwefelatom, $K^+$ ein Kation der Formel

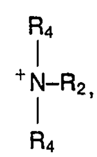,

$R_2$ Wasserstoff, $C_1$–$C_3$-Hydroxyalkyl, Methyl oder Ethyl, $R_4$ Wasserstoff oder Methyl oder Ethyl und $A^-$ für den Fall der fluorierten Gruppen unter der Bedeutung von R ein $C_5$–$C_8$-n-Alkyl- oder n-Alkenylsulfonat-Anion, ein Trifluormethyl- oder Pentafluorethylsulfonat-Anion, ein $C_6$–$C_8$-n-Alkyl- oder n-Alkenylcarboxylat-Anion, ein $C_1$–$C_4$-n-Perfluoralkyl oder $C_1$–$C_4$-n-Perfluoralkenylcarboxylat-Anion, ein Anion der Formel

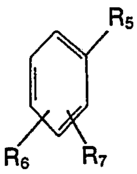

oder

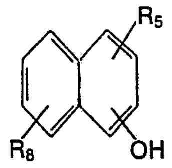

$R_5$–$COO^-$ oder $-SO_3^-$, $R_6$ $C_1$–$C_5$-Alkyl, $C_2$–$C_5$-Alkenyl oder $C_1$–$C_5$-Alkoxy in den Stellungen 3, 4 oder 5 zu $R_5$, oder $R_6$ Wasserstoff, wenn $R_7$ Hydroxy ist, $R_7$ Wasserstoff oder Hydroxy in den Stellungen 2 oder 3 zu $R_5$ oder $R_7$ $NO_2^-$, $F^-$, $Cl^-$, $Br^-$ oder $J^-$ in der Stellung 3 zu $R_5$, $R_8$ Wasserstoff oder Methyl, oder $A^-$ ein Rhodanid-Anion oder ein Anion der Formel $C_2Cl_{2n+1}COO^-$ mit n = 1, 2 oder 3 und $A^-$ für den Fall der nicht-fluorierten Gruppen unter der Bedeutung von R ein n-Fluoralkyl- oder n-Fluoralkenyl-carboxylat-Anion, wobei die Summe von (2x + n) eine ganze Zahl von 19 bis 24 betragen soll, wenn x die Anzahl der C-Atome im Carboxylat-Anion und n die Anzahl der C-Atome in der Gruppe R ist; ein n-Fluoralkyl- oder n-Fluoralkenyl-sulfonat-Anion, wobei die Summe von (2y + n) 17 oder 18 betragen soll, wenn y die Anzahl der C-Atome im Sulfonat-Anion und n die Anzahl der C-Atome in der Gruppe R ist.

3. Quartäre Ammoniumverbindungen der Formel $R$–$K^+A$ nach Anspruch 1, wobei $R$–$K^+$ ein Kation der Formel

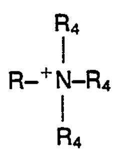

oder

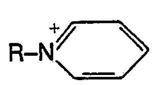

R eine Gruppe der Formeln $C_nF_{2n+1}$–$C_aH_{2a}$–, $C_nF_{2n}$–$C_{2a-1}$–, $C_nF_{2n+1}$–$CONR_1C_kH_{2k}$– oder $C_nF_{2n+1}$–$C_2H_4SC_kH_{2k}$–, $C_nF_{2n+1}SO_2NR_1C_kH_{2k}$–,

n eine Zahl von 2 bis 10, a eine Zahl von 2 bis 20, k eine Zahl von 2 bis 6, wobei die Summe (2n + a) und (2n + k) eine Zahl von 12 bis 24 betragen soll, $R_4$ Methyl oder Ethyl, $R_4'$ Methyl, Ethyl, Hydroxymethyl oder Hydroxyethyl, $R_1$ Methyl oder Ethyl, $A^-$ ein 2-Hydroxyphenylsulfonat-, m-Halogenbenzoat- oder Salicylat-Anion, ein Anion der Formel

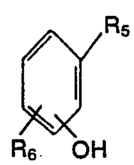

worin $R_5$ $COO^-$ oder $SO_3^-$ und $R_6$ $C_nH_{2n+1}$– oder $C_nH_{2n}$– oder $C_nH_{2n+1}$–O– mit n einer Zahl von 1 bis 4 in den Stellungen 3, 4 oder 5 zu $R_5$ bedeuten oder $A^-$ eine 2-Hydroxy-1-naphthoat-, 3- oder 4-Hydroxy-2-naphthoat-, 2-Hydroxy-naphthalin-1-sulfonat-, 3- oder 4-Hydroxy-naphthalin-2-sulfonat-Anion, oder $A^-$ ein Anion der Formel $CF_3SO_3^-$ oder n–$C_xH_{2x+1}$–$SO_3^-$, wobei x 6 ist für den Fall (2n + a) mindestens 18, x ist 7 für den Fall (2n + a) mindestens 16 oder X ist 8 für den Fall (2n + a) eine Zahl von 14 bis 18 oder $A^-$ ist ein Anion der Formel n–$C_xF_{2x+1}$–$COO^-$, wobei x 2 oder 3 ist, wenn (2n + a) eine Zahl von 16 bis 22 ist oder X ist 4 oder 5, wenn (2n + a) eine Zahl von 14 bis 20 ist oder x ist eine Zahl von 6 bis 9, wenn (2n + a) eine Zahl von 14 bis 22 ist, oder $A^-$ ein Anion der Formeln $SCN^-$ oder $CCl_3COO^-$ bedeutet für den Fall n mindestens 6 und (2n + 1) mindestens 16.

4. Quartäre Ammoniumverbindungen der Formel $R$–$K^+A^-$, wobei $R$–$K^+$ ein Kation der Formel

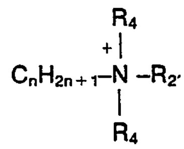

oder

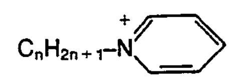

n eine Zahl von 1 bis 22, $R_4$ Methyl oder Ethyl, $R_2'$ Wasserstoff, Methyl, Ethyl, Hydroxymethyl oder Hydroxyethyl, $A^-$ ein Anion der Formeln n–$C_2F_{2x+1}$–$COO^-$, wobei x 2 oder 3 ist, wenn n + 2x eine Zahl von 20 bis 28 ist, oder x ist 4 oder 5, wenn n + 2x eine Zahl von 20 bis 26 ist, oder x ist eine Zahl von 6 bis 9, wenn (n + x) eine Zahl von 19 bis 22 ist, oder $A^-$ ein Anion der Formel n–$C_xF_{2x+1}$–$SO_3^-$ bedeutet, wobei x eine Zahl ist, die so gewählt ist, dass die Summe (2x + n) 17 oder 18 beträgt.

5. Verwendung von quaternären Ammoniumverbindungen der Formel

$$R\text{–}K^+A^-$$

als Strömungsbeschleuniger, wobei R n-Fluoralkyl, n-Fluoralkenyl, n-Alkyl, n-Alkenyl oder eine Gruppe der Formeln

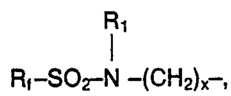

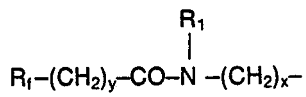

oder

$$R_f\text{–}(CH_2)_yB(CH_2)_x\text{–}$$

$R_f$ n-Fluoralkyl oder n-Fluoralkenyl, $R_1$ Wasserstoff, Methyl oder Ethyl, X eine ganze Zahl von 2 bis 6, y 0, 1 oder 2, B ein Sauerstoff- oder Schwefelatom,

$K^+$ ein Kation der Formeln

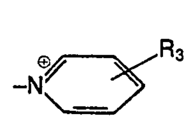

oder

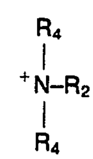

$R_2$ Wasserstoff, $C_1$–$C_4$-Hydroxyalkyl, $C_1$–$C_5$-Alkyl, Benzyl oder Phenyl, $R_3$ Wasserstoff, Methyl oder Ethyl, $R_4$ jeweils gleich oder verschieden sein können, Wasserstoff oder $C_1$–$C_3$-Alkyl und $A^-$ für den Fall der fluorierten Gruppen unter der Bedeutung von R ein n-Alkyl-, n-Alkenyl- oder n-Fluoralkylsulfonat-Anion, ein

n-Alkyl-, n-Alkenyl- oder n-Fluoralkenylcarboxylat-Anion, ein Benzoat-, Phenylsulfonat-, Naphthoat-, Jodid-, Rhodanid-Anion oder ein Anion der Formel $C_nCl_{2n+1}COO^-$ mit n = 1, 2 oder 3 und $A^-$ für den Fall der nichtfluorierten Gruppe unter der Bedeutung von R ein n-Alkyl-, n-Alkenylcarboxylat-Anion, ein n-Fluoralkyl- oder

n-Fluoralkenylcarboxylat-Anion oder ein n-Fluoralkylsulfonat- oder n-Fluoralkenylsulfonat-Anion bedeuten, wobei die Verbindungen ausgenommen sind für die Fälle R = Fluoralkyl und $K^+$ Trialkylammonium und $A^-$ = Jodid; R = Alkyl und $K^+ = N^+H_3$ und $A^-$ = Fluoralkylcarboxylat und $R\text{-}K^+$ = $(C_1\text{-}C_2)$-Trialkylammonium, $A^-$ = Fluoralkylsulfonat und Fluoralkylcarboxylat, ebenso die Salze der Formeln

$$[C_{16}H_{33}N(CH_3)_3]^+\ C_nH_{2n+1}COO^-$$

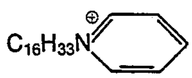 $C_nH_{2n+1}COO^-$,

$[C_nH_{2n+1}$ 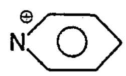 $]\ C_xF_{2x+1}COO^-$

für n = 12–14
und x = 1–3.

## Claims

1. A quaternary ammonium compound of the formula

$$R\text{-}K^+A^-$$

in which R denotes n-fluoroalkyl, n-fluoroalkenyl, n-alkyl, n-alkenyl or a group of the formula

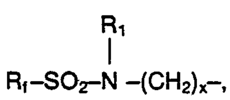 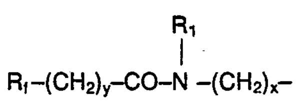

or

$$R_f\text{-}(CH_2)_yB(CH_2)_x\text{-}$$

$R_f$ denotes n-fluoroalkyl or n-fluoroalkenyl, $R_1$ denotes hydrogen, methyl or ethyl, x denotes an integer from 2 to 6, y denotes 0, 1 or 2, B denotes an oxygen or sulfur atom, $K^+$ denotes a cation of the formula

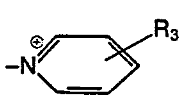 or 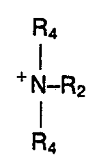

$R_2$ denotes hydrogen, $C_1$–$C_4$-hydroxyalkyl, $C_1$–$C_5$-alkyl, benzyl or phenyl, $R_3$ denotes hydrogen, methyl or ethyl, the radicals $R_4$, which can be identical or different, in each case denote hydrogen or $C_1$–$C_3$-alkyl and, in the case of the fluorinated groups R, $A^-$ denotes an n-alkyl-, n-alkenyl- or n-fluoroalkyl-sulfonate anion, an n-alkyl-, n-alkenyl- or n-fluoroalkenyl-carboxylate anion, a benzoate, phenylsulfonate, naphthoate or thiocyanate anion or an anion of the formula $C_n Cl_{2n+1} COO^-$, where n = 1, 2 or 3, or, in the case of the non-fluorinated groups R, $A^-$ denotes an n-fluoroalkyl- or n-fluoroalkenyl-carboxylate anion or an n-fluoroalkylsulfonate or n-fluoroalkenylsulfonate anion, the compounds being excluded in the cases where R = fluoroalkyl, fluoroalkenyl and $K^+$ = trialkylammonium and $A^-$ = alkylsulfonate, alkenylsulfonate, phenylsulfonate, acetate; R = alkyl, $K^+ = N^+H_3$ and $A^-$ = fluoroalkylcarboxylate; and $R\text{-}K^+$ = $(C_1\text{-}C_2)$-trialkylammonium and $A^-$ = fluoroalkylsulfonate or fluoroalkylcarboxylate; and also the salts of the formula

$[C_nH_{2n+1}$ 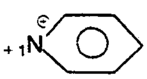 $]\ C_xF_{2x+1}COO^\ominus$

where n = 12–14 and x = 1–3.

2. A quaternary ammonium compound of the formula $R\text{-}K^+A^-$, in which R denotes $C_8$–$C_{14}$-n-fluoroalkyl, $C_8$–$C_{14}$-n-fluoroalkenyl, $C_1$–$C_{24}$-n-alkyl or $C_1$–$C_{24}$-n-alkenyl, or a group of the formula

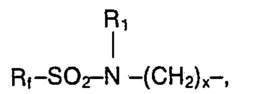 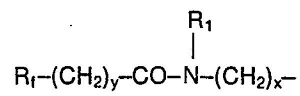

or

$$R_f\text{-}(CH_2)_yB(CH_2)_x\text{-}$$

$R_f$ denotes $C_8$–$C_{14}$-n-fluoroalkyl or $C_8$–$C_{14}$-n-fluoroalkenyl

$R_1$ denotes methyl or ethyl, x denotes an integer from 2 to 6, y denotes 0, 1 or 2, B denotes an oxygen or sulfur atom, $K^+$ denotes a cation of the formula

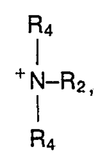

$R_2$ denotes hydrogen, $C_1$–$C_3$-hydroxyalkyl, methyl or ethyl, $R_3$ denotes hydrogen, $R_4$ denotes hydrogen, methyl or ethyl and, in the case of the fluorinated groups R, $A^-$ denotes a $C_5$–$C_8$-n-alkyl- or -n-alkenyl-sulfonate anion, a trifluoromethyl- or pentafluoroethyl-sulfonate anion, a $C_6$–$C_8$-n-alkyl- or -n-alkenyl-carboxylate anion, a $C_1$–$C_4$-n-perfluoroalkyl- or $C_1$–$C_4$-n-perfluoroalkenyl-carboxylate anion, or an anion of the formula

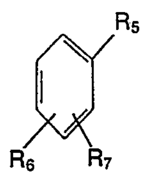 or 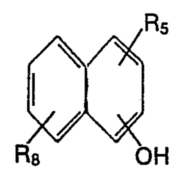

in which $R_5$ denotes $-COO^-$ or $-SO_3^-$, $R_6$ denotes $C_1$–$C_5$-alkyl, $C_2$–$C_5$-alkenyl or $C_1$–$C_5$-alkoxy in position 3, 4 or 5 relative to $R_5$, or $R_6$ denotes hydrogen, if $R_7$ is hydroxyl, $R_7$ denotes hydrogen or hydroxyl in position 2 or 3 relative to $R_5$, or $R_7$ denotes $NO_2^-$, $F^-$, $Cl^-$, $Br^-$ or $I^-$ in position 3 relative to $R_5$, and $R_8$ denotes hydrogen or methyl, or $A^-$ denotes a thiocyanate anion or an anion of the formula $C_2Cl_{2n+1}COO^-$, where n = 1, 2 or 3, or, in the case of non-fluorinated groups R, $A^-$ denotes an n-fluoroalkyl- or n-fluoroalkenyl-carboxylate anion, and the sum of (2x + n) should be an integer from 19 to 24, if x is the number of carbon atoms in the carboxylate anion and n is the number of carbon atoms in the group R; or an n-fluoroalkyl- or n-fluoroalkenyl-sulfonate anion, and the sum of (2y + n) should be 17 or 18, if y is the number of carbon atoms in the sulfonate anion and n is the number of carbon atoms in the group R.

3. A quaternary ammonium compound of the formula $R–K^+A$ as claimed in claim 1, in which $R–K^+$ denotes a cation of the formula

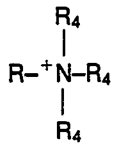 or 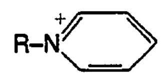

R denotes a group of the formula $C_nF_{2n+1}–C_aH_{2a}–$, $C_nF_{2n}–C_{2a-1}–$, $C_nF_{2n+1}–CONR_1C_kH_{2k}–$ or $C_nF_{2n+1}–C_2H_4SC_kH_{2k}–$, $C_nF_{2n+1}\ SO_2NR_1C_kH_{2k}–$,

n denotes a number from 2 to 10, a denotes a number from 2 to 20, and k denotes a number from 2 to 6, and the sum of (2n+a) and (2n+k) should be a number from 12 to 24,

$R_4$ denotes methyl or ethyl, $R_4'$ denotes methyl, ethyl, hydroxymethyl or hydroxyethyl, $R_1$ denotes methyl or ethyl, and

$A^-$ denotes a 2-hydroxyphenylsulfonate, m-halogenobenzoate or salicylate anion, an anion of the formula

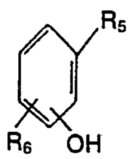

in which $R_5$ denotes $COO^-$ or $SO_3^-$ and $R_6$ denotes $C_nH_{2n+1}–$, $C_nH_{2n}–$ or $C_nH_{2n+1}–O–$, where n is a number from 1 to 4, in position 3, 4 or 5 relative to $R_5$, or $A^-$ denotes a 2-hydroxy-1-naphthoate, 3- or 4-hydroxy-2-naphthoate, 2-hydroxy-naphthalene-1-sulfonate or 3- or 4-hydroxy-naphthalene-2-sulfonate anion, or $A^-$ denotes an anion of the formula $CF_3SO_3^-$ or $n–C_xH_{2x+1}–SO_3^-$, in which x is 6 if (2n+a) is at least 18, x is 7 if (2n+a) is at least 16 or x is 8 if (2n+a) is a number from 14 to 18, or $A^-$ is an anion of the formula $n–C_xF_{2x+1}–COO^-$, in which x is 2 or 3, if (2n+a) is a number from 16 to 22, or x is 4 or 5, if (2n+a) is a number from 14 to 20, or x is a number from 6 to 9, if (2n+a) is a number from 14 to 22, or $A^-$ denotes an anion of the formula $SCN^-$ or $CCl_3COO^-$, if n is at least 6 and (2n+1) is at least 16.

4. A quaternary ammonium compound of the formula $R–K^+A^-$, in which $R–K^+$ is a cation of the formula

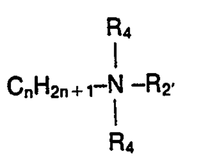 or 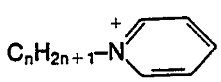

n denotes a number from 1 to 22, $R_4$ denotes methyl or ethyl, $R_2'$ denotes hydrogen, methyl, ethyl, hydroxymethyl or hydroxyethyl and $A^-$ denotes an anion of the formula $n–C_2F_{2x+1}–COO^-$, in which x is 2 or 3, if n+2x is a number from 20 to 28, or x is 4 or 5, if n+2x is a number from 20 to 26, or x is a number from 6 to 9, if (n+x) is a number from 19 to 22, or $A^-$ denotes an anion of the formula $n–C_xF_{2x+1}–SO_3^-$, in which x is a number which is chosen so that the sum (2x+n) is 17 or 18.

5. Use of a quaternary ammonium compound as claimed in claim 1 of the formula

$$R–K^+A^-$$

as a drag agent in which R denotes n-fluoroalkyl, n-fluoroalkenyl, n-alkyl, n-alkenyl or a group of the formula

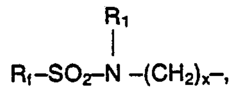, 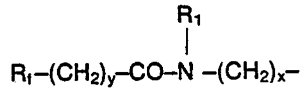

or

$$R_f–(CH_2)_yB(CH_2)_x–$$

$R_f$ denotes n-fluoroalkyl or n-fluoroalkenyl, $R_1$ denotes hydrogen, methyl or ethyl, x denotes an integer from 2 to 6, y denotes 0, 1 or 2, B denotes an oxygen or sulfur atom, $K^+$ denotes a cation of the formula

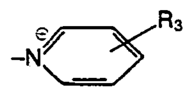 or 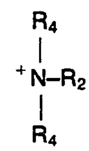

$R_2$ denotes hydrogen, $C_1–C_4$-hydroxyalkyl, $C_1–C_5$-alkyl, benzyl or phenyl, $R_3$ denotes hydrogen, methyl or ethyl, the radicals $R_4$, which can be identical or different, in each case denote hydrogen or $C_1–C_3$-alkyl and, in the case of the fluorinated groups R, $A^-$ denotes an n-alkyl-, n-alkenyl- or n-fluoroalkyl-sulfonate anion, an n-alkyl-, n-alkenyl- or n-fluoroalkenyl-carboxylate anion, a benzoate, phenylsulfonate, naphthoate, iodide or thiocyanate anion or an anion of the formula $C_nCl_{2n+1}COO^-$, where n = 1, 2 or 3, or, in the case of the non-fluorinated groups R, $A^-$ denotes an n-alkyl- or n-alkenyl-carboxylate anion, an n-fluoroalkyl- or n-fluoroalkenyl-carboxylate anion or an n-fluoroalkylsulfonate or n-fluoroalkenylsulfonate anion, the compounds being excluded in the cases where R = fluoroalkyl, $K^+$ = trialkylammonium and $A^-$ = iodide; R = alkyl, $K^+ = N^+H_3$ and $A^-$ = fluoroalkylcarboxylate; and $R–K^+ = (C_1–C_2)$-trialkylammonium and $A^-$ = fluoroalkylsulfonate or fluoroalkylcarboxylate; and also the salts of the formulae $[C_{16}H_{33}N(CH_3)_3]^+\ C_nH_{2n+1}COO^-$,

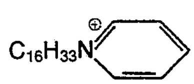 $C_nH_{2n+1}COO^-$, $C_nH_{2+1}COO^-$

and

$[C_nH_{2n+1}$ 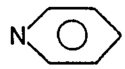 $]\ C_xF_{2x+1}COO^-$

where n = 12–14 and x = 1–3.

## Revendications

1. Composés d'ammoniums quaternaires qui répondent à la formule:

$$R\text{–}K^+A^-$$

dans laquelle:

R représente un radical fluoro-n-alkyle, fluoro-n-alcényle, n-alkyle ou n-alcényle ou un radical répondant à l'une des formules suivantes:

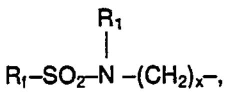 , 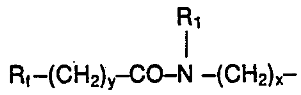

et

$$R_f\text{–}(CH_2)_yB(CH_2)_x\text{–}$$

$R_f$ représente un radical fluoro-n-alkyle ou fluoro-n-alcényle, $R_1$ l'hydrogène, un méthyle ou un éthyle, x un nombre entier de 2 à 6, y un nombre égal à 0, à 1 ou à 2, B un atome d'oxygène ou de soufre,

$K^+$ représente un cation répondant à l'une des formules:

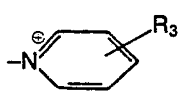 et 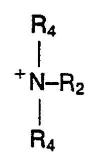

$R_2$ représente l'hydrogène, un hydroxy-alkyle en $C_1$–$C_4$, un alkyle en $C_1$–$C_5$, un benzyle ou un phényle, $R_3$ l'hydrogène, un méthyle ou un éthyle, les $R_4$ représentent chacun, indépendamment l'un de l'autre, l'hydrogène ou un alkyle en $C_1$–$C_3$, et

$A^-$ représente:

dans le cas où R est un radical fluoré:

un anion n-alkyl-sulfonate, n-alcényl-sulfonate, fluoro-n-alkyl-sulfonate, n-alkyl-carboxylate, n-alcényl-carboxylate, fluoro-n-alcényl-carboxylate, benzoate, phénylsulfonate, naphtoate ou thiocyanate ou un anion $C_nCl_{2n+1}COO^-$ dans lequel n est égal à 1, 2 ou à 3, et

dans le cas où R est un radical non fluoré: un anion fluoro-n-alkyl-carboxylate, fluoro-n-alcényl-carboxylate, fluoro-n-alkyl-sulfonate ou fluoro-n-alcényl-sulfonate,

à l'exception des composés dans lesquels R représente un fluoralkyle ou un fluoralcényle, $K^+$ un trialkylammonium et $A^-$ un alkylsulfonate, un alcénylsulfonate, un phénylsulfonate ou un acétate, de ceux dans lesquels R représente un alkyle, $K^+$ représente $N^+H_3$ et $A^-$ un fluoralkylcarboxylate, et de ceux dans lesquels R–$K^+$ représente un trialkylammonium dont chacun des alkyles contient un ou deux atomes de carbone et $A^-$ représente un fluoralkyl-sulfonate ou un fluoralkyl-carboxylate, ainsi que des sels qui répondent à la formule:

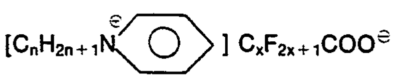

dans laquelle n désigne un nombre de 12 à 14 et x un nombre de 1 à 3.

2. Composés d'ammoniums quaternaires répondant à la formule:

$$R\text{–}K^+A^-$$

dans laquelle:

R représente un fluoro-n-alkyle en $C_8$–$C_{14}$, un fluoro-n-alcényle en $C_8$–$C_{14}$, un n-alkyle en $C_1$–$C_{24}$, un n-alcényle en $C_1$–$C_{24}$ ou un radical répondant à l'une des formules suivantes:

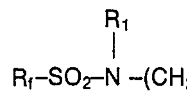 , 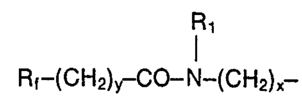

et

$$R_f\text{–}(CH_2)_yB(CH_2)_x\text{–}$$

$R_f$ représente un fluoro-n-alkyle en $C_8$–$C_{14}$ ou un fluoro-n-alcényle en $C_8$–$C_{14}$, $R_1$ un méthyle ou un éthyle, x un nombre entier de 2 à 6, y est égal à 0, à 1 ou à 2, B représente un atome d'oxygène ou de soufre,

$K^+$ représente un cation de formule:

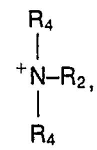

dans lequel $R_2$ représente l'hydrogène, un hydroxy-alkyle en $C_1$–$C_3$, un méthyle ou un éthyle, $R_3$ l'hydrogène et $R_4$ l'hydrogène, un méthyle ou un éthyle, et

$A^-$ représente:

dans le cas où R est un radical fluoré: un anion n-alkyl-sulfonate en $C_5$–$C_8$, n-alcényl-sulfonate, trifluorométhyl-sulfonate, pentafluoréthyl-sulfonate, n-alkyl-carboxylate en $C_6$–$C_8$, n-alcényl-carboxylate, n-perfluoralkyl-carboxylate en $C_1$–$C_4$ ou n-perfluoralcényl-carboxylate en $C_1$–$C_4$ ou un anion répondant à l'une des formules:

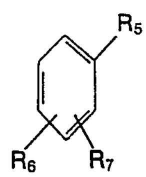 et 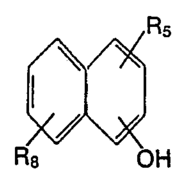

$R_5$ représente $-COO^-$ $-SO_3^-$, $R_6$ représente un alkyle en $C_1$–$C_5$, un alcényle en $C_2$–$C_5$ ou un alcoxy en $C_1$–$C_5$ occupant l'une des positions 3, 4 et 5 par rapport à $R_5$ ou encore $R_6$ représente l'hydrogène lorsque $R_7$ est un hydroxy, $R_7$ représente l'hydrogène ou un hydroxy occupant l'une des positions 2 et 3 par rapport à $R_5$, ou $R_7$ représente $NO_2^-$, $F^-$, $Cl^-$, $Br^-$ ou $I^-$ en position 3 par rapport à $R_5$, $R_8$ représente l'hydrogène ou un méthyle, ou $A^-$ représente un anion thiocyanate ou un anion $C_nCl_{2n+1}COO^-$ dans lequel n est égal à 1, à 2 ou à 3, et

dans le cas où R est un radical non fluoré: un anion fluoro-n-alkyl-carboxylate ou fluoro-n-alcényl-carboxylate, la somme (2x + n) dans laquelle x est le nombre des atomes de carbone de l'anion carboxylate et n celui du radical R devant être un nombre entier de 19 à 24, ou un anion fluoro-n-alkyl-sulfonate ou fluoro-n-alcényl-sulfonate, la somme (2y + n) dans laquelle y est le nombre des atomes de carbone de l'anion sulfonate et n celui du radical R devant être égal à 17 ou à 18.

3. Composés d'ammoniums quaternaires de formule $R{-}K^+A^-$ selon la revendication 1 dans lesquels $R{-}K^+$ représente un cation répondant à l'une des formules:

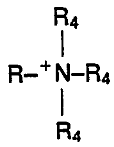
et
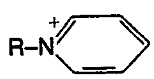

R représente un radical $C_nF_{2n+1}{-}C_aH_{2a}{-}$, $C_nF_{2n}{-}C_{2a-1}{-}$, $C_nF_{2n+1}{-}CONR_1C_kH_{2k}{-}$, $C_nF_{2n+1}{-}C_2H_4SC_kH_{2k}{-}$ ou $C_nF_{2n+1}\ SO_2NR_1C_kH_{2k}{-}$, n désigne un nombre de 2 à 10, a un nombre de 2 à 20, k un nombre de 2 à 6, les sommes (2n+a) et (2n+k) devant chacune être égale à un nombre de 12 à 24, $R_4$ représente un méthyle ou un éthyle, $R_4'$ un méthyle, un éthyle, un hydroxyméthyle ou un hydroxy-éthyle, $R_1$ un méthyle ou un éthyle et

$A^-$ représente un anion hydroxy-2 phényl-sulfonate, m-halogéno-benzoate ou salicylate ou un anion répondant à la formule:

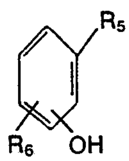

dans laquelle $R_5$ représente $COO^-$ ou $SO_3^-$ et $R_6$ un radical $C_nH_{2n+1}{-}$, $C_nH_{2n}{-}$ ou $C_nH_{2n+1}{-}O{-}$ (l'indice n désigne ici un nombre de 1 à 4) occupant l'une des positions 3, 4 et 5 par rapport à $R_5$, ou

$A^-$ représente un anion hydroxy-2 naphtoate, hydroxy-3 ou -4 naphtoate-2, hydroxy-2 naphtalène-sulfonate-1, hydroxy-3 ou -4 naphtalène-sulfonate-2, ou

$A^-$ représente un anion $CF_3SO_3^-$ ou un anion $n{-}C_xH_{2+1}{-}SO_3^-$ dans lequel x est égal à 6 lorsque (2n+a) est au moins égal à 18, x est égal à 7 lorsque (2n+a) est au moins égal à 16, et x est égal à 8 lorsque (2n+a) est un nombre de 14 à 18, ou

$A^-$ représente un anion $n{-}C_xF_{2x+1}{-}COO^-$ dans lequel x est égal à 2 ou à 3 lorsque (2n+a) est un nombre de 16 à 22, ou x est égal à 4 ou à 5 lorsque (2n+a) est un nombre de 14 à 20, ou x est un nombre de 6 à 9 lorsque (2n+a) est un nombre de 14 à 22, ou

$A^-$ représente un anion $SCN^-$ ou $CCl_3COO^-$ lorsque n est au moins égal à 6 et que (2n+1) est au moins égal à 16.

4. Composés d'ammoniums quaternaires répondant à la formule:

$$R{-}K^+A^-$$

dans laquelle:

$R{-}K^+$ représente un cation répondant à l'une des formules:

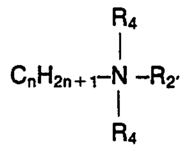
et
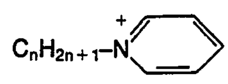

n désigne un nombre de 1 à 22, $R_4$ représente un méthyle ou un éthyle, $R_2'$ représente l'hydrogène ou un radical méthyle, éthyle, hydroxyméthyle ou hydroxy-éthyle, et

$A^-$ représente un anion $n{-}C_xF_{2x+1}{-}COO^-$ dans lequel x est égal à 2 ou à 3 lorsque (n+2x) est un nombre de 20 à 28, ou x est égal à 4 ou à 5 lorsque (n+2x) est un nombre de 20 à 26, ou x est un nombre de 6 à 9 lorsque (n+x) est un nombre de 19 à 22, ou

$A^-$ représente un anion $n{-}C_xF_{2x+1}{-}SO_3^-$ dans lequel x est un nombre tel que la somme (2x+n) soit égale à 17 ou à 18.

5. Application, comme accélérateurs d'écoulement, de composés d'ammoniums quaternaires répondant à la formule:

$$R{-}K^+A^-$$

dans laquelle:

R représente un radical fluoro-n-alkyle, fluoro-n-alcényle, n-alkyle ou n-alcényle ou un radical répondant à l'une des formules suivantes:

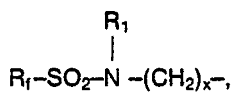
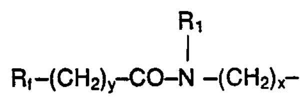

et

$$R_f{-}(CH_2)_yB(CH_2)_x{-}$$

$R_f$ représente un radical fluoro-n-alkyle ou fluoro-n-alcényle, $R_1$ l'hydrogène, un méthyle ou un éthyle, x un nombre entier de 2 à 6, y un nombre égal à 0, à 1 ou à 2, B un atome d'oxygène ou de soufre,

$K^+$ représente un cation répondant à l'une des formules:

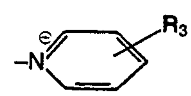
et
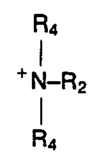

$R_2$ représente l'hydrogène, un hydroxy-alkyle en $C_1$–$C_4$, un alkyle en $C_1$–$C_5$, un benzyle ou un phényle, $R_3$ l'hydrogène, un méthyle ou un éthyle, les $R_4$ représentent chacun, indépendamment l'un de l'autre, l'hydrogène ou un alkyle en $C_1$–$C_3$, et

$A^-$ représente:

dans le cas où R est un radical fluoré: un anion n-alkyl-sulfonate, n-alcényl-sulfonate, n-fluoralkyl-sulfonate, n-alkyl-carboxylate, n-alcényl-carboxylate, n-fluoralcényl-carboxylate, benzoate, phénylsulfonate, naphtoate, iodure ou thiocyanate ou un anion $C_nCl_{2n+1}\ COO^-$ dans lequel n est égal à 1, à 2 ou à 3, et

dans le cas où R est un radical non fluoré: un anion n-alkyl-carboxylate, n-alcényl-carboxylate, fluoro-n-alkyl-carboxylate, fluoro-n-alcényl-carboxylate, fluoro-n-alkyl-sulfonate ou fluoro-n-alcényl-sulfonate,

à l'exception des composés dans lesquels R représente un radical fluoralkyle, $K^+$ un trialkylammonium et $A^-$ un ion iodure, de ceux dans lesquels R représente un radical alkyle, $K^+$ représente $N^+H_3$ et $A^-$ représente un ion fluoralkylcarboxylate, et de ceux dans lesquels R–$K^+$ représente un trialkylammonium dont chacun des alkyles contient 1 ou 2 atomes de carbone, $A^-$ représente un ion fluoralkyl-sulfonate ou fluoralkylcarboxylate, ainsi que des sels répondant aux formules:

$$[C_{16}H_{33}N(CH_3)_3]^+ \ C_nH_{2n+1}COO^-,$$

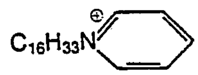 $C_nH_{2n+1}COO^-$,

$[C_nH_{2n+1}$ 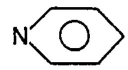 $]\ C_xF_{2x+1}COO^-$

et $C_nH_{2+1}COO^-$

dans lesquelles n désigne un nombre de 12 à 14 et x un nombre de 1 à 3.

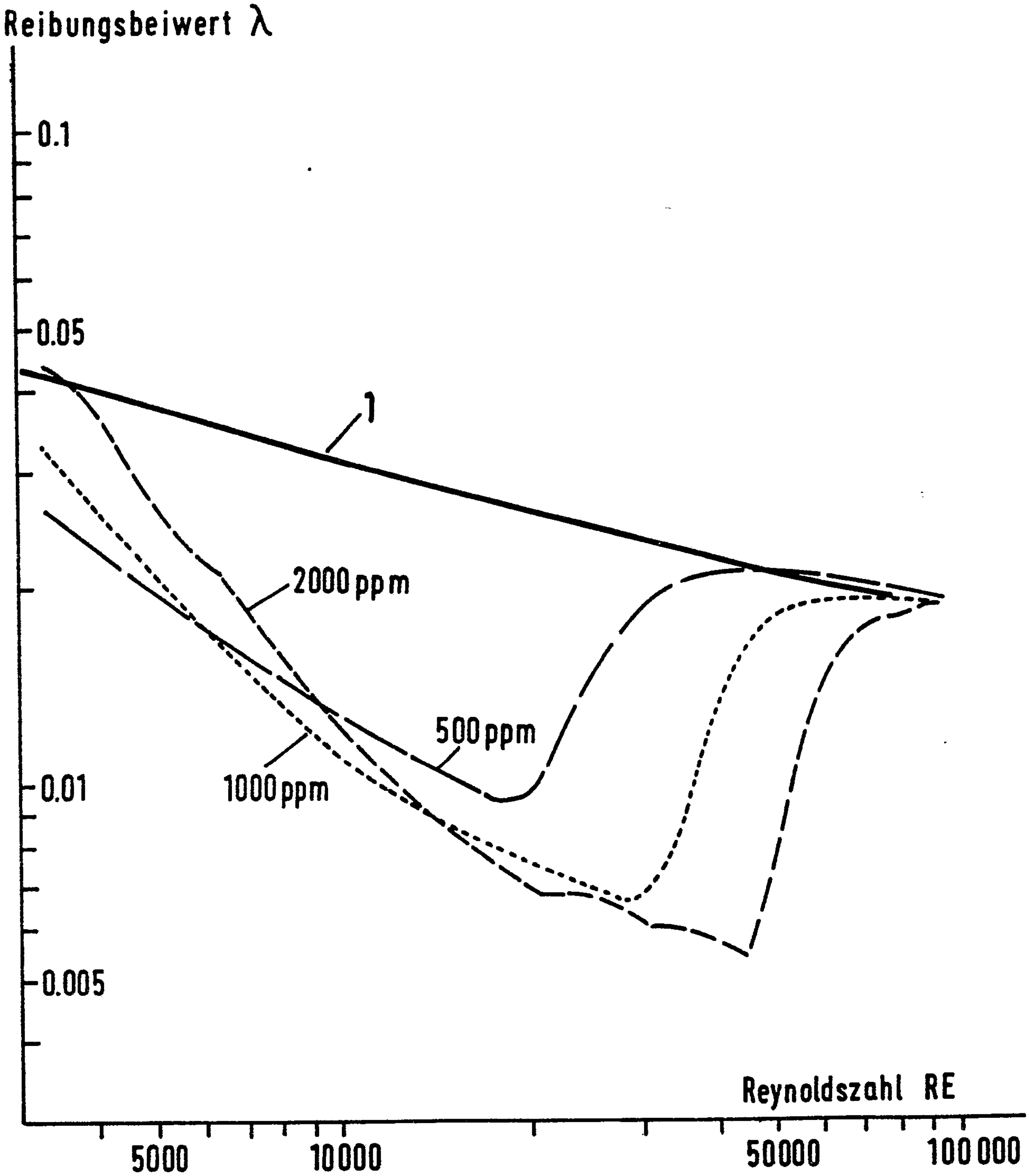
Reibungsbeiwert λ
0.1
0.05
0.01
0.005
1
2000 ppm
500 ppm
1000 ppm
Reynoldszahl RE
5000
10000
50000
100 000